(19) Europäisches Patentamt
European Patent Office
Office européen des brevets

(11) **EP 4 304 692 B1**

(12) **EUROPEAN PATENT SPECIFICATION**

(45) Date of publication and mention
of the grant of the patent:
**29.04.2026 Bulletin 2026/18**

(21) Application number: **22766493.5**

(22) Date of filing: **10.03.2022**

(51) International Patent Classification (IPC):
*A61M 16/16* (2006.01)     *A61M 16/10* (2006.01)
*A61M 16/20* (2006.01)     *G05D 16/16* (2006.01)
*G05D 7/06* (2006.01)      *A61M 16/00* (2006.01)

(52) Cooperative Patent Classification (CPC):
(C-Sets available)
**A61M 16/026; A61M 16/0069;** A61M 16/125;
A61M 16/16; A61M 2016/0039; A61M 2016/1025;
A61M 2205/18; A61M 2205/3365; A61M 2205/3368;
A61M 2205/505; A61M 2230/205          (Cont.)

(86) International application number:
**PCT/IB2022/052131**

(87) International publication number:
**WO 2022/190020 (15.09.2022 Gazette 2022/37)**

(54) **A RESPIRATORY APPARATUS**

ATEMVORRICHTUNG

APPAREIL RESPIRATOIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.03.2021 US 202163160285 P**

(43) Date of publication of application:
**17.01.2024 Bulletin 2024/03**

(60) Divisional application:
**26164424.9**

(73) Proprietor: **Fisher & Paykel Healthcare Limited
2013 Auckland (NZ)**

(72) Inventor: **BURGESS, Russel William
Auckland, 2013 (NZ)**

(74) Representative: **Forresters IP LLP
Skygarden
Erika-Mann-Straße 11
80636 München (DE)**

(56) References cited:
WO-A1-2007/019628     WO-A1-2020/171720
US-A1- 2008 163 872    US-A1- 2012 248 636
US-A1- 2013 042 869    US-A1- 2015 007 821
US-A1- 2019 151 583    US-A1- 2020 179 629
US-A1- 2020 405 984

(52) Cooperative Patent Classification (CPC): (Cont.)

C-Sets
A61M 2230/205, A61M 2230/005

**Description**

**FIELD OF THE DISCLOSURE**

The present disclosure relates to an apparatus and control methods for providing respiratory support and/or therapy using a flow of gases.

**BACKGROUND**

Respiratory apparatuses are used in various environments such as hospital, medical facility, residential care, or home environments to deliver a flow of gas to users or patients. A respiratory apparatus, for example a flow therapy apparatus, may include an oxygen inlet to allow delivery of supplemental oxygen with the flow of gas, and/or a humidification apparatus to deliver heated and humidified gases. A flow therapy apparatus may allow adjustment and control over characteristics of the gases flow, including flow rate, temperature, gas concentration, such as oxygen concentration, humidity, pressure, etc.

US patent application publication 2008/0163872 discloses a device for administering a breathing gas at alternating pressure levels, including taking into account thermal load.

**SUMMARY**

The present invention provides a respiratory support apparatus, as also defined in the appended claims, that is configured to provide a flow of gases to a user for respiratory flow therapy comprising: a flow generator that is operable to generate a flow of gases; a controller that is operatively connected to the flow generator and operable to control a flow rate of the flow of gases by controlling a motor speed of the flow generator, wherein the controller is configured to: receive an input that represents a set flow rate at which the apparatus is to generate the flow of gases for a desired therapy session comprising respiratory flow therapy; predict if a steady-state temperature of the apparatus will exceed a high-temperature-condition threshold, based at least partly on a motor speed required to generate the set flow rate; and generate a predicted-high-temperature-condition signal if the high-temperature-condition threshold is exceeded based on the prediction.

In a configuration, predicting if the steady-state temperature will exceed the high-temperature-condition threshold is based on determining if the required motor speed exceeds a maximum allowable motor speed, wherein the maximum allowable motor speed represents the highest motor speed, for the set flow rate, that the apparatus can sustain without the steady-state temperature exceeding the high-temperature-condition threshold.

In a configuration, the maximum allowable motor speed is based on, or is a function of, a predetermined heat model.

In a configuration, the heat model represents the heat generated by the flow generator, which is a function of the flow rate and the motor speed.

In a configuration, the heat model represents the heat generated by the flow generator, which is a function of the flow rate and the motor speed, and one or more non-flow generator heat sources.

In a configuration, the heat model is configured to represent the one or more non-flow generator heat sources with respective preset values representing an estimate of the maximum heat that could be generated by each of the one or more non-flow generator heat sources during operation.

In a configuration, the heat model is configured to represent the one or more non-flow generator heat sources with one or more respective predetermined or configurable safety values that represent the maximum heat predicted to be generated by the respective heat sources in the desired therapy session.

In a configuration, the maximum allowable motor speed for a range of flow rates is represented or defined by a predetermined threshold line plotted on a motor speed versus flow rate graph, wherein the area above the threshold line represents a high-temperature-condition zone.

In a configuration, predicting if the steady-state temperature will exceed the high-temperature-condition threshold is based on determining if the required motor speed and set flow rate combination is in the high-temperature-condition zone.

In a configuration, predicting if the steady-state temperature will exceed the high-temperature-condition threshold is based on: predicting the steady-state temperature as a function of the required motor speed and set flow rate; and determining if the predicted steady-state temperature exceeds the high-temperature-condition threshold.

In a configuration, predicting the steady-state temperature comprises determining an estimate from a predetermined heat model.

In a configuration, the heat model represents the heat generated by the flow generator, which is a function of the flow rate and the motor speed.

In a configuration, the heat model represents the heat generated by the flow generator, which is a function of the flow rate and the motor speed, and one or more non-flow generator heat sources.

In a configuration, the heat model is configured to represent the one or more non-flow generator heat sources with respective preset values representing an estimate of the maximum heat that could be generated by each of the one or more non-flow generator heat sources during operation.

In a configuration, the heat model is configured to represent the one or more non-flow generator heat sources with one or more respective predetermined or configurable safety values that represent the maximum heat predicted to be generated by the respective heat sources in the desired therapy session.

In a configuration, the controller is configured to predict if the steady-state temperature of the apparatus will exceed the high-temperature-condition threshold by: determining if the required motor speed exceeds a maximum allowable motor speed for the set flow rate, the maximum allowable motor speed representing the high-temperature-condition threshold.

In a configuration, the maximum allowable motor speed for the set flow rate is determined based on, or is a function of, a predetermined heat model.

In a configuration, the heat model is a function or threshold line representing the maximum allowable motor speed for a range of set flow rates, and wherein the heat model is generated at least partly based on the high-temperature-condition threshold.

In a configuration, the controller is configured to predict if the steady-state temperature of the apparatus will exceed the high-temperature-condition threshold by: predicting the steady-state temperature based on, or as a function of, a predetermined heat model, the set flow rate, and the required motor speed; and determining if the predicted steady-state temperature exceeds the high-temperature-condition threshold.

In a configuration, the heat model generates a temperature variable representing the predicted steady-state temperature based at least on the set flow rate and the required motor speed such that the heat model is at least based on the heat generated by the flow generator.

In a configuration, the heat model generates a temperature variable representing the predicted steady-state temperature based at least on the set flow rate, the required motor speed, and one or more other parameters representing non-flow generator heat-sources of or associated with the apparatus that will contribute to the steady-state temperature of the apparatus.

In a configuration, the controller is configured to determine if the temperature variable representing the predicted steady-state temperature exceeds the high-temperature-condition threshold.

In a configuration, the heat model represents one or more heat sources of or associated with the apparatus, including at least representing the heat generated by the flow generator as a function of motor speed and flow rate.

In a configuration, the heat model further represents the heat generated by one or more non-flow generator heat sources of or associated with the apparatus that will contribute to the steady-state temperature of the apparatus.

In a configuration, the heat model is based at least partly on a theoretical heat model of the apparatus.

In a configuration, the heat model is based at least partly on experimental heat data associated with operation of the apparatus.

In a configuration, the heat model is based at least partly on one or more predetermined or configurable safety values.

In a configuration, the controller is configured to determine the required motor speed of the flow generator to generate the set flow rate.

In a configuration, the controller is configured to determine the required motor speed to generate the set flow rate by increasing or changing the motor speed until the set flow rate is generated by the flow generator.

In a configuration, the controller is configured to receive the set flow rate input for the desired therapy session when the apparatus is at start-up or standby with the flow generator stopped or idling at a non-therapy speed prior to the desired therapy session beginning.

In a configuration, the controller is configured to receive the set flow rate input while the apparatus is already operating in a current therapy session at a first flow rate, and the set flow rate input is a different second flow rate relating to the new desired therapy session.

In a configuration, the controller is configured to predict if the steady-state temperature of the apparatus will exceed the high-temperature-condition threshold based at least partly on the required motor speed by: estimating a first heat value representing the heat contributing to the steady-state temperature by one or more non-flow generator heat sources; estimating a maximum allowable motor speed of the flow generator corresponding to a second heat value representing the heat contributing to the steady-state temperature by the flow generator such that the aggregate of the first heat value and second heat value do not cause the steady-state temperature to exceed the high-temperature-condition threshold; increasing or changing the motor speed of the flow generator to the motor speed required for the flow generator to generate the set flow rate so as to thereby determine the required motor speed for that set flow rate; and predicting that the steady-state temperature of the apparatus will exceed the high-temperature-condition threshold if the determined required motor speed is above the estimated maximum allowable motor speed.

In a configuration, the controller is configured to predict if the steady-state temperature of the apparatus will exceed a high-temperature-condition threshold based at least partly on the required motor speed by: increasing or changing the motor speed of the flow generator to the motor speed required for the flow generator to generate the set flow rate so as to thereby determine the required motor speed for that set flow rate; estimating a first steady-state temperature value representing the heat contributed to the steady-state temperate by the flow generator; estimating a second steady-state temperature value representing the heat contributed to the steady-state temperature by one or more non-flow generator heat sources; predicting the steady-state temperature of the apparatus by aggregating the first steady-state temperature value and second steady-state temperature value; and evaluating if the predicted steady-state temperature exceeds the high-temperature-condition threshold.

In a configuration, the one or more non-flow generator heat sources comprise any one or more of the following non-flow generator components or variables of, connected to, or associated with the apparatus: ambient temperature of environment surrounding apparatus, a battery or power source, a heater plate of a humidifier, a heater wire of a patient interface conduit, electronic circuitry or components, a Printed Circuit Board (PCB), a graphical user interface, and/or a display screen.

In a configuration, the steady-state temperature is configurable to represent any predictable temperature of interest of, within, or associated with the apparatus.

In a configuration, the steady-state temperature represents a sustained and/or stable temperature parameter, as opposed to a transient temperature parameter.

In a configuration, the steady-state temperature represents a predicted temperature parameter after the apparatus has been operating in the desired therapy session for at least a predetermined minimum time period at the set flow rate such that the steady-state temperature represents a sustained and/or stable temperature parameter.

In a configuration, the steady-state temperature of the apparatus represents the predicted temperature of the flow of gases in the apparatus at a specified region or location within a gases flow path of the apparatus.

In a configuration, the steady-state temperature of the apparatus represents the predicted temperature within a specific location or region of a main housing of the apparatus.

In a configuration, the steady-state temperature of the apparatus represents the predicted temperature at or in the region of a specific component or componentry of the respiratory support apparatus.

In a configuration, the steady-state temperature of the apparatus represents a predicted generic overall temperature parameter associated with the apparatus based on a heat model of the apparatus and/or one or more heat sources of interest of or associated with the apparatus.

In a configuration, the high-temperature-condition threshold is a configurable temperature parameter.

In a configuration, the high-temperature-condition threshold is a temperature value.

In a configuration, the high-temperature-condition threshold is a temperature parameter that is predetermined or selected based on the characteristics of the apparatus and/or the specific type of steady-state temperature being predicted.

In a configuration, the steady-state temperature represents a predicted temperature parameter relating to the temperature of flow of gases at a specified region or location within the apparatus, and the high-temperature-condition threshold is configured to represent a gases temperature safety threshold or value above which there is an increased risk of faulty operation, damage to the apparatus, and/or a safety risk to the user.

In a configuration, the steady-state temperature represents a predicted temperature parameter relating to a specific location or region of a main housing of the apparatus, and the high-temperature-condition threshold is configured to represent a housing temperature safety threshold or value above which there is an increased risk of faulty operation, damage to the apparatus, and/or a safety risk to the user.

In a configuration, the controller is configured to generate an alarm if a predicted-high-temperature-condition signal is generated.

In a configuration, the alarm comprises any one of more of the following types of alarms: audible, visual, tactile, and/or haptic.

In a configuration, the alarm comprises an alarm message or indication on a display screen or a user interface of the apparatus.

In a configuration, the controller is configured to reject the set flow rate and disable the motor or reduce the motor to an idling speed if the alarm is generated.

In a configuration, the controller is configured to cap the motor speed to a maximum allowable motor speed and to generate an alarm message or indication on a display screen or a user interface of the apparatus if an alarm is generated, and wherein the maximum allowable motor speed represents the highest motor speed, for the set flow rate, that the apparatus can sustain without the steady-state temperature exceeding the high-temperature-condition threshold.

In a configuration, the controller is configured to reduce or disable the power applied to or operation of one or more non-flow generator components of the apparatus to lower their heat output if the alarm is generated.

In a configuration, the controller is configured to reduce the power applied to and/or disable a heater plate of a humidifier and/or a heater wire of a patient interface conduit if the alarm is generated.

In a configuration, the controller is configured to reduce or disable the charging circuitry associated with a battery of the apparatus if the alarm is generated.

In a configuration, the steady-state temperature and high-temperature-condition threshold are represented and determined with reference to a temperature metric or a thermal or heat energy metric.

In a configuration, the flow generator comprises one or more thermistors located within the housing of the flow generator or in thermal communication with the flow generator or a motor of the flow generator, and wherein the thermistor(s) are configured to sense a temperature of or relating to the flow generator and/or motor of the flow generator, and generate representative temperature signal(s).

In a configuration, the apparatus further comprises a humidifier.

In a configuration, the humidifier comprises a humidification chamber (e.g. a humidification water chamber). In a configuration, the humidification chamber may be removable from the apparatus.

In a configuration, the humidifier comprises a heater plate or heater component or heater element that is configured to heat the water in the chamber.

In a configuration, the apparatus further comprises a main housing. In a configuration, the main housing comprises the flow generator and the humidifier. In one example, the flow generator and the humidifier may be integrated into or provided in the same or a common main housing. For example, the humidifier may comprise the humidification chamber and heater plate.

In a configuration, the main housing of the apparatus comprises the flow generator and the humidifier (e.g. humidification chamber and heater plate), and one or more other heat sources of the apparatus. For example, the one or more other heat sources may comprise any one or more or all of: a battery or power source, a heater wire, electronic circuity or components, Printer Circuit Board or Boards (PCBs), a graphical user interface, and/or display system. In one configuration, the main housing comprises or provides the flow generator, the humidifier, a battery or power source, and all other heat sources of the apparatus. For example, all the heat generating components may be provided or integrated into a common main housing.

Also disclosed, but not independently claimed, is a method of controlling a respiratory support apparatus that is configured to provide a flow of gases to a user for respiratory therapy, the apparatus comprising: a flow generator that is operable to generate a flow of gases; a controller that is operatively connected to the flow generator and operable to control a flow rate of the flow of gases by controlling a motor speed of the flow generator, wherein the method is executable or implemented by the controller and comprises: receiving an input that represents a set flow rate at which the apparatus is to generate the flow of gases for a desired therapy session; predicting if a steady-state temperature of the apparatus will exceed a high-temperature-condition threshold, based at least partly on a motor speed required to generate the set flow rate; and generating a predicted-high-temperature-condition signal if the high-temperature-condition threshold is exceeded based on the prediction.

Also disclosed, but not independently claimed, is a respiratory support apparatus that is configured to provide a flow of gases to a user for respiratory therapy comprising: a flow generator that is operable to generate a flow of gases; a controller that is operatively connected to the flow generator and operable to control one or more properties of the flow of gases provided to the user by controlling a motor speed of the flow generator, wherein the controller is configured to: receive one or more input therapy settings that represents one or more properties desired for the generated flow of gases provided to a user during a therapy session; predict one or more motor speed limits, based at least partly on the one or more input or therapy settings and a heat model of the apparatus, that will prevent a steady-state temperature of the apparatus from exceeding a high-temperature-condition threshold; and controlling the flow generator toward providing the flow of gases according to or based on the input therapy settings to the extent possible while operating within the motor speed limit(s).

In a configuration, the input therapy setting(s) comprise a set flow rate indicative of the desired flow rate of the gases provided to the user for a flow therapy session.

In a configuration, the input therapy setting(s) comprise one or more pressure settings relating to the desired pressure of the flow of gases provided to the user for a positive airway pressure therapy session.

In a configuration, the controller controls the flow generator to deliver the flow of gases in full compliance with the therapy settings while operating within the motor speed limit(s).

In a configuration, the controller controls the flow generator to deliver the flow of gases to a reduced level relative to the input therapy settings due to operating within the motor speed limit(s).

Also disclosed, but not independently claimed, is a method of controlling a respiratory support apparatus that is configured to provide a flow of gases to a user for respiratory therapy, the apparatus comprising: a flow generator that is operable to generate a flow of gases; a controller that is operatively connected to the flow generator and operable to control one or more properties of the flow of gases provided to the user by controlling a motor speed of the flow generator, wherein the method is executable or implemented by the controller and comprises: receiving one or more input therapy settings that represents one or more properties desired for the generated flow of gases provided to a user during a therapy session;

predicting one or more motor speed limits, based at least partly on the one or more input or therapy settings and a heat model of the apparatus, that will prevent a steady-state temperature of the apparatus from exceeding a high-temperature-condition threshold; and controlling the flow generator toward providing the flow of gases according to or based on the input therapy settings to the extent possible while operating within the motor speed limit(s).

Also disclosed, but not independently claimed, is a respiratory support apparatus that is configured to provide a flow of gases to a user for respiratory therapy comprising: a flow generator that is operable to generate a flow of gases; a controller that is operatively connected to the flow generator and operable to control one or more properties of the flow of gases provided to the user by controlling a motor speed of the flow generator, wherein the controller is configured to: receive one or more input therapy settings that represents one or more properties desired for the generated flow of gases provided to a user during a therapy session; predict one or more motor speed limits, based at least partly on the one or more input or therapy settings and a heat model of the apparatus, that will prevent a steady-state temperature of the apparatus from exceeding a high-temperature-condition threshold; control the flow generator to provide the flow of gases according to the input therapy settings; and alarm and/or initiate an alarm response counteraction control measure if the motor speed of the flow generator is operating outside of the motor speed limits for a predetermined time period to prevent or reduce the risk of the apparatus exceeding the high-temperature-condition threshold.

Also disclosed, but not independently claimed, is a method of controlling a respiratory support apparatus that is configured to provide a flow of gases to a user for respiratory therapy, the apparatus comprising: a flow generator that is operable to generate a flow of gases; a controller that is operatively connected to the flow generator and operable to control one or more properties of the flow of gases provided to the user by controlling a motor speed of the flow generator, wherein the method is executable or implemented by the controller and comprises: receiving one or more input therapy settings that represents one or more properties desired for the generated flow of gases provided to a user during a therapy session; predicting one or more motor speed limits, based at least partly on the one or more input or therapy settings and a heat model of the apparatus, that will prevent a steady-state temperature of the apparatus from exceeding a high-temperature-condition threshold; controlling the flow generator to provide the flow of gases according to the input therapy settings; and alarming and/or initiating an alarm response counteraction control measure if the motor speed of the flow generator is operating outside of the motor speed limits for a predetermined time period to prevent or reduce the risk of the apparatus exceeding the high-temperature-condition threshold.

Also disclosed, but not independently claimed, is a respiratory support apparatus that is configured to provide a flow of gases to a user for respiratory therapy comprising: a flow generator that is operable to generate a flow of gases; a controller that is operatively connected to the flow generator and operable to control one or more properties of the flow of gases provided to the user by controlling a motor speed of the flow generator, wherein the controller is configured to: receive one or more input therapy settings that represents one or more properties desired for the generated flow of gases provided to a user during a therapy session; predict if a steady-state temperature of the apparatus will exceed a high-temperature-condition threshold, based at least partly on one or more of the input therapy settings and a heat model of the apparatus; and generate an alarm and/or initiate an alarm response counteraction control measure in response to the prediction to prevent or reduce the risk of the apparatus exceeding the high-temperature-condition threshold.

In a configuration, the input therapy setting(s) comprise a set flow rate indicative of the desired flow rate of the gases provided to the user for a flow therapy session.

In a configuration, the input therapy setting(s) comprise one or more pressure settings relating to the desired pressure of the flow of gases provided to the user for a positive airway pressure therapy session.

Also disclosed, but not independently claimed, is a method of controlling a respiratory support apparatus that is configured to provide a flow of gases to a user for respiratory therapy, the apparatus comprising: a flow generator that is operable to generate a flow of gases; a controller that is operatively connected to the flow generator and operable to control one or more properties of the flow of gases provided to the user by controlling a motor speed of the flow generator, wherein the method is executable or implemented by the controller and comprises: receiving one or more input therapy settings that represents one or more properties desired for the generated flow of gases provided to a user during a therapy session; predicting if a steady-state temperature of the apparatus will exceed a high-temperature-condition threshold, based at least partly on one or more of the input therapy settings and a heat model of the apparatus; and generating an alarm and/or initiating an alarm response counteraction control measure in response to the prediction to prevent or reduce the risk of the apparatus exceeding the high-temperature-condition threshold.

Also disclosed, but not independently claimed, is a respiratory support apparatus that is configured to provide a flow of gases to a user for respiratory therapy comprising: a flow generator that is operable to generate a flow of gases; a controller that is configured to control the flow generator by controlling a motor speed of the flow generator, wherein the controller is configured to: receive an input that represents a set flow rate at which the apparatus is to generate the flow of gases for a therapy session; determine a motor speed of the flow generator that corresponds to achieving the set flow rate; and activating a visual and/or audible alarm if the determined motor speed of the flow generator for the set flow rate is predicted to cause a steady-state temperature of the apparatus to exceed a predetermined high-temperature condition threshold.

In a configuration, the visual alarm is presented as an alarm notification or message on a display screen of the apparatus.

In a configuration, the steady-state temperature is a prediction of a temperature of or within the apparatus in the future, and wherein the steady-state temperature is calculated or predicted based at least partly on the determined motor speed.

In a configuration, the steady-state temperature is predicted based on the determined motor speed and a heat model of the apparatus.

In a configuration, the heat model defines the predicted temperature increase to the steady-state temperature of the apparatus caused by one or more selected non-flow generator heat source components operating in the apparatus.

In a configuration, the heat model defines the predicted temperature increase to the steady-state temperature of the apparatus caused by the flow generator operating at the determined motor speed and one or more selected non-flow generator heat source components operating in the apparatus.

Also disclosed, but not independently claimed, is a method of controlling a respiratory support apparatus that is configured to provide a flow of gases to a user for respiratory therapy, the apparatus comprising: a flow generator that is operable to generate a flow of gases; a controller that is configured to control the flow generator by controlling a motor speed of the flow generator, wherein the method is executable or implemented by the controller and comprises: receiving an input that represents a set flow rate at which the apparatus is to generate the flow of gases for a therapy session; determining a motor speed of the flow generator that corresponds to achieving the set flow rate; and activating a visual and/or audible alarm if the determined motor speed of the flow generator for the set flow rate is predicted to cause a steady-state temperature of the apparatus to exceed a predetermined high-temperature condition threshold.

Also disclosed, but not independently claimed, is a respiratory support apparatus that is configured to provide a flow of gases to a user for respiratory therapy comprising: a flow generator that is operable to generate a flow of gases; a controller that is operatively connected to the flow generator and operable to control a flow rate of the flow of gases by controlling a motor speed of the flow generator, wherein the controller is configured to: receive an input that represents a set flow rate at which the apparatus is to generate the flow of gases for a desired therapy session; determine the motor speed of the flow generator required to generate the set flow rate; and raise an alarm associated with a potential future high-temperature condition of the apparatus, the alarm being raised within a predetermined time and/or before an actual steady-state temperature of the apparatus exceeds a temperature threshold or limit for the set flow rate.

In a configuration, the predetermined time is less than the duration of a therapy session.

In a configuration, the predetermined time is less than 30 minutes from the commencement of the therapy session.

In a configuration, the predetermined time is less than 15 minutes from the commencement of the therapy session.

In a configuration, the predetermined time is less than a plurality of seconds from the commencement of the therapy session.

In a configuration, the predetermined time comprises the time prior to a preset time period before the actual steady-state temperature of the apparatus exceeds the temperature threshold or limit, such that the controller is configured to raise the alarm at a time which is at least the preset time period before the actual steady-state temperature of the apparatus exceeds the temperature threshold or limit.

In a configuration, the controller is configured to increase or ramp-up the motor speed of the flow generator toward the set motor speed at the commencement of the therapy session, and wherein the predetermined time for raising the alarm comprises the time period of the motor speed ramping-up to the set motor speed or the time period after the motor speed has reached the set motor speed.

In a configuration, the controller is configured to raise the alarm if the actual steady-state temperature of the apparatus increases in a manner that will cause a potential future high-temperature condition of the apparatus.

In a configuration, the controller is configured to: predict if the steady-state temperature of the apparatus will exceed the temperature threshold or limit, based at least partly on the motor speed required to generate the set flow rate; and raise the alarm if the predicted steady-state temperature of the apparatus exceeds the temperature threshold or limit.

In a configuration, the controller is configured to present an alarm notification or message on a display of the apparatus when the alarm is raised.

In a configuration, the alarm notification comprises a message indicating that the current set flow rate will cause the actual steady-state temperature of the apparatus to exceed a temperature threshold or limit.

In a configuration, the controller is configured to: predict a maximum steady-state temperature of the apparatus based on a heat model and at least the motor speed required for the set flow rate; compare the predicted maximum steady-state temperature of the apparatus to the temperature threshold or limit; and raise the alarm if the predicted maximum steady-state temperature exceeds the temperature threshold or limit.

In a configuration, the heat model represents the heat generated by the flow generator, which is a function of the flow rate and the motor speed.

In a configuration, the heat model represents the heat generated by the flow generator, which is a function of the flow rate and the motor speed, and one or more non-flow generator heat sources.

In a configuration, the controller is configured to predict the maximum steady-state temperature of the apparatus within the predetermined time.

In a configuration, if the alarm is raised, the controller is configured to action any one or more of the following: switching off the flow generator; reducing power applied to a heater plate of a humidifier of the apparatus; reducing power applied to a heater wire of a patient circuit conduit of or connected to the apparatus; reducing power applied to the heater plate and heater wire; and/or presenting an audible and/or alphanumeric or visual message on a display of the apparatus.

Also disclosed, but not independently claimed, is a method of controlling a respiratory support apparatus that is configured to provide a flow of gases to a user for respiratory therapy, the apparatus comprising: a flow generator that is operable to generate a flow of gases; a controller that is operatively connected to the flow generator and operable to control a flow rate of the flow of gases by controlling a motor speed of the flow generator, wherein the method is executable or implemented by the controller and comprises: receiving an input that represents a set flow rate at which the apparatus is to generate the flow of gases for a desired therapy session; determining the motor speed of the flow generator required to generate the set flow rate; and raising an alarm associated with a potential future high-temperature condition of the apparatus, the alarm being raised within a predetermined time and/or before an actual steady-state temperature of the apparatus exceeds a temperature threshold or limit for the set flow rate.

Also disclosed, but not independently claimed, is a non-transitory computer-readable medium having stored thereon computer executable instructions that, when executed on a processing device or devices, cause the processing device or devices to perform a method of any one of the previous aspects of the disclosure.

Any one or more of the features and/or configurations mentioned in respect of one aspect of the disclosure above may be applied to any one or more of the other aspects of the disclosure above.

## Definitions or terms or phrases

The phrase 'computer-readable medium' should be taken to include a single medium or multiple media. Examples of multiple media include a centralised or distributed database and/or associated caches. These multiple media store the one or more sets of computer executable instructions. The phrase 'computer readable medium' should also be taken to include any medium that is capable of storing, encoding or carrying a set of instructions for execution by a processor of a computing device and that cause the processor to perform any one or more of the methods described herein. The computer-readable medium is also capable of storing, encoding or carrying data structures used by or associated with these sets of instructions. The phrase 'computer-readable medium' includes solid-state memories, optical media and magnetic media.

The term "comprising" as used in this specification and claims means "consisting at least in part of". When interpreting each statement in this specification and claims that includes the term "comprising", features other than that or those prefaced by the term may also be present. Related terms such as "comprise" and "comprises" are to be interpreted in the same manner.

The phrase 'respiratory apparatus' as used in this specification and claims is intended to mean, unless the context suggests otherwise, any type or form of apparatus, machine, system or device, that is configured to provide and/or generate a flow of gases to a user for the purpose of respiratory support, respiratory therapy, and including apparatus which provide one or more different operational modes and types of support or therapy, whether flow-controlled, pressure-controlled, or otherwise, and including apparatus configured or operable to provide flow therapy, high-flow therapy, oxygen therapy, positive airway pressure (PAP) therapies such as, but not limited to, continuous positive airway pressure (CPAP) therapy or bi-level CPAP (BiPAP) therapy, and the phrases 'flow therapy apparatus', 'respiratory therapy apparatus', 'respiratory support apparatus', 'breathing assistance apparatus' may be used to refer to a type of 'respiratory apparatus', depending on the context.

The phrase 'flow generator' as used in this specification and claims is intended to mean, unless the context suggests otherwise, any device, system, component, or configuration that is configured to generate a pressurised flow of gases including, but not limited to, a blower having an impeller driven by a motor, compressor, or other pressurised source of gases.

The phrase 'temperature' as used in this specification and claims is intended to mean, unless the context suggests otherwise, a measure or parameter representing heat or thermal energy and may be expressed in degrees Celsius (C), degrees Fahrenheit (F), or Kelvin (K), or any equivalent, and is also intended to capture any indirect associated measures or metrics related to temperature, such as direct measures of heat or thermal energy in metrics such as British thermal units (Btu) or joules or similar, such that references to 'temperature' in the context of the predictive alarm of the disclosure and as claimed is intended to cover direct temperature metrics, and/or heat or thermal energy metrics. For example, reference to a temperature exceeding a threshold can be determined based on temperature metrics or heat or thermal energy metrics, such that the predictive alarm can be implemented in the temperature metric domain or energy metric domain. Additionally, in this context, reference to 'heat model' need not necessarily be restricted to a model based on heat or thermal energy, but can be considered to also include or be equivalent to a 'temperature model' which is based on a temperature metric or similar. Likewise, the phrases 'over-temperature' and 'over-heat' or variants of these, may be used interchangeably.

The phrase 'therapy session' as used in this specification and claims is intended to mean, unless the context suggests otherwise, a period of operation of the respiratory apparatus, which may have a start and end. A therapy session may have or be defined by a preset or predetermined time period or may have a configurable time period defined by a time metric. Each time period may be being defined by hours and/or minutes for example. A therapy session may additionally or alternatively have a start and/or end that is defined by a target objective or metric measured or assessed by a sensed operating parameter of the device or sensed physiological parameter of the user. A therapy session may also be an adhoc session of random or arbitrary duration. A 'therapy session' is generally intended to mean a session of use of the respiratory apparatus for a particular, specified, or prescribed respiratory therapy and/or use of the apparatus for general respiratory support or other use generally.

The phrase "predict" as used in this specification and claims are intended to mean, unless the context suggests otherwise, determining or calculating, whether directly or indirectly, an estimate or expectation relating to a future state, condition, and/or value of or associated with a parameter, variable, and/or aspect of an apparatus. The phrases, terms, and/or descriptors "predictive", "predicting", and "predicted" are used in the same general sense, unless the context suggests otherwise.

## Prior Art

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

## Number Ranges

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

As used herein the term "and/or" means "and" or "or", or both.

As used herein "(s)" following a noun means the plural and/or singular forms of the noun.

This disclosure consists in the foregoing and also envisages constructions of which the following gives examples only.

## BRIEF DESCRIPTION OF THE DRAWINGS

**Figure 1A** shows in diagrammatic form a flow therapy apparatus;

**Figure 1B** shows a sensing circuit board including a flow rate sensor that may be used in a flow therapy apparatus;

**Figure 2** is a flow diagram showing an overview process of a predictive over-temperature alarm of the disclosure;

**Figure 3** is a flow diagram showing an overview of the process of a predictive over-temperature alarm of the disclosure in accordance with a first embodiment;

**Figure 4A** is a graph of motor speed versus flow rate, and demonstrating a maximum allowable motor speed curve in the context of the first embodiment based on a blower-only heat model;

**Figure 4B** shows the graph of Figure 4A with an alternative maximum allowable motor speed curve in the context of the first embodiment based on an alternative more complete heat model taking into account the blower heat source and one or more other non-blower heat sources;

**Figure 4C** is a flow diagram showing a decision tree implemented by the predictive over-temperature alarm of the first embodiment;

**Figure 5** is a flow diagram showing an overview of the process of a predictive over-temperature alarm of the disclosure in accordance with a second embodiment;

**Figure 6A** is a graph of motor speed versus flow rate, and demonstrating a steady-state temperature for a motor speed-flow rate combination compared against a threshold curve for a heat model used in the context of the second embodiment; and

**Figure 6B** is a flow diagram showing a decision tree implemented by the predictive over-temperature alarm of the second embodiment;

## DETAILED DESCRIPTION

In the following description, specific details are given to provide a thorough understanding of the embodiments. However, it will be understood by one of ordinary skill in the art that the embodiments may be practiced without these specific details. For example, software modules, functions, circuits, etc., may be shown in block diagrams in order not to obscure the embodiments in unnecessary detail. In other instances, well-known modules, structures and techniques may not be shown in detail in order not to obscure the embodiments.

Also, it is noted that the embodiments may be described as a process that is depicted as a flowchart, a flow diagram, a structure diagram, or a block diagram. Although a flowchart may describe the operations as a sequential process, many of the operations can be performed in parallel or concurrently. In addition, the order of the operations may be rearranged. A process is terminated when its operations are completed. A process may correspond to a method, a function, a procedure, a subroutine, a subprogram, etc., in a computer program. When a process corresponds to a function, its termination corresponds to a return of the function to the calling function or a main function.

## 1. <u>Overview</u>

Respiratory apparatus and devices can overheat under certain conditions. This overheating can be caused by heat generated by components within the apparatus or device such as, but not limited to: flow generator (e.g. a blower), power sources such as a battery, heating element or heater plate of humidifier, heater wire of patient interface conduit, electronic circuitry and/or components including those provided on Printed Circuit Boards (PCBs), display screens and/or graphical user interface, and other such components. Alternatively or additionally, overheating in respiratory devices can arise from other heat sources such as the ambient environment and the heated and/or humidified gases flowing through the device.

Some respiratory apparatuses have temperature sensors that sense the temperature of one or more components. If the sensed or measured temperature of the one or more components or the temperature of the gases provided to the patient exceeds a threshold, the apparatus alarms. One problem with such existing temperature alarms is related to the time between the therapy session starting and the apparatus alarming. In a common use case, a nurse (or the patient, or other user) will connect a certain interface (e.g. mask or cannula for example) to the patient and the apparatus, input certain device settings, and begin the therapy session. In a hospital context, the nurse will then move on to other tasks, only to have the temperature alarm activate some time (e.g. many minutes or even over an hour) later. The nurse will then have to return to the patient, make changes to the apparatus settings, and restart the therapy session. Having to return to a patient who has already completed part of a therapy session can be frustrating for nurses (who are often time-poor) and also to the patient that has experienced an interruption to respiratory therapy or support. Similar issues exist in a homecare context. For example, a patient or user may start or initiate a therapy session on the respiratory apparatus at home, and then may typically start or continue with other tasks such as reading, watching media on devices, and/or other recreational activities or home-based tasks. The user or patient's therapy and recreational or other activity is then disrupted if the temperature alarm triggers many minutes or an hour or more later, and then need to re-configure and restart the therapy session, which is frustrating. Further, if the temperature alarm is activated in either a hospital or homecare context, the nurse and/or patient may need to wait for the apparatus to cool down to allow acceptable operation again, and this can cause further delay to re-starting the respiratory therapy or support. Another problem with existing temperature alarms is that the alarm does not trigger until the sensed temperature crosses a threshold temperature, and this exposes or allows the device and/or particular components of the device to experience some level of exposure to high temperature or heating, which can damage the components and/or reduce their usable lifetime and/or affect their accuracy or operation ability.

The present disclosure provides a respiratory apparatus with a control system and method that implements a predictive maximum-device-temperature alarm (also referred to herein as a 'predictive over-temperature alarm') that activates if the input or operational therapy settings to the apparatus for the start of a therapy session will likely cause the steady-state temperature of the apparatus to exceed a high-temperature-condition-threshold. The predictive maximum-device-temperature alarm may also operate periodically, continuously, or may be initiated in response to a change in operating conditions (e.g. a user or patient or nurse or clinician changing the input or operational settings) during the therapy session to re-check or check if the current, new or changed input or operational settings will likely cause a steady-state temperature of the apparatus to exceed the high-temperature-condition-threshold.

In the context of a hospital or hospice environment, the predictive alarm can be configured to activate at or soon after the therapy session has commenced, which is likely before the nurse has moved on from the patient, and/or before a user or patient has commenced a recreational activity or other task of the nature discussed above. A predictive alarm may therefore save nurses from having to wait for the device to actually exceed its maximum steady-state temperature before alarming, thereby reducing the likelihood of them having to return to a patient who has already completed part of a therapy session. In the context of a homecare environment in which a user or patient is using the respiratory apparatus at home, the predictive alarm can alarm early (e.g. a few seconds or a few minutes after therapy settings are selected) in a therapy session to warn the user that the selected therapy settings or operating conditions will cause the respiratory apparatus to exceed a high-temperature-condition-threshold (i.e. overheat condition). The user or homecare patient can then change or modify the therapy settings in response to the predictive alarm to thereby minimise potential future interruptions or over-temperature alarms during the therapy session caused by overheating of the apparatus.

Additionally, the predictive alarm of the present disclosure further safeguards the device and/or its components from excessive temperatures or overheating, as the alarm is triggered based on a predicted future steady-state temperature of or associated with the device exceeding a high-temperature-condition threshold, and therefore triggers before the device and/or components are exposed to undesirable high temperatures or overheating.

Embodiments of the predictive over-temperature alarm of this disclosure will primarily be described in the context of a respiratory apparatus in the form of a flow therapy apparatus of the type explained further below. An embodiment of the predictive over-temperature alarm will also be described in the context of a pressure therapy apparatus. However, it will be appreciated that the predictive over-temperature alarm could be adapted and implemented in any suitable respiratory apparatus that is configured to deliver a flow of gases to a user for respiratory therapy or support, where the apparatus comprises or is exposed to heat sources that may cause an unsafe over-temperature condition. The predictive alarm can be applied to any respiratory apparatus that delivers any one or more types of respiratory therapy and/or support including, but not limited to, flow therapy, high-flow therapy, oxygen therapy, positive airway pressure (PAP) therapies such as continuous positive airway pressure (CPAP) or Bi-level positive airway pressure (BiPAP) or the like. Such respiratory apparatus typically comprise a controllable flow generator (e.g. blower or compressor) and optionally a humidifier. Depending on the particular respiratory therapy or support being provided, the respiratory apparatus can deliver the flow of gases according to set input flow rates or pressure values or settings, and via a sealed or unsealed interface (e.g. full-face mask, nasal mask, oral mask, nasal cannula or the like). For example, the predictive over-temperature alarm can be adapted to predict over-temperature conditions based on or according to the input or therapy settings applicable to the particular type of respiratory therapy or support being provided, whether flow rate settings, pressure-level settings, and/or any other relevant therapy settings (such as, but not limited to, therapy duration time period, humidification settings, gases temperature settings, oxygen concentration settings for example).

It will be appreciated that the predictive over-temperature alarm can be applied to a respiratory apparatus that is capable of delivering more than one type of respiratory therapy and/or operating in more than one therapy mode. In one example, the respiratory apparatus may be operable to deliver flow therapy (e.g. high flow therapy) or pressure therapy (e.g. BiLevel pressure therapy or CPAP therapy), depending on the mode of operation initiated. As will be appreciated, the controller of such a respiratory apparatus may control the flow rate of the gases stream when providing flow therapy or support, and may control the pressure when providing pressure therapy or support. For flow therapies, typically the gases stream generated is delivered to the patient by a patient circuit comprising a conduit or tube and an unsealed interface (e.g. nasal cannula). For pressure therapies, typically the gases stream is delivered to the patient by a patient circuit comprising a conduit or tube and a sealed interface (e.g. a full face mask or nasal mask). In some configurations, the same type of conduit or tube may be used in both flow and pressure therapy modes. The patient circuit may be connected to or part of the respiratory apparatus.

## 2. Respiratory Apparatus

By way of example only, various embodiments of the predictive over-temperature alarm will be described in the context of the following flow therapy apparatus, but the application of the alarm is not limited to such configurations and apparatus, as explained above. The flow therapy apparatus described below is to provide context to further understand the implementation and operation of the predictive over-temperature alarm. It will be appreciated that a flow therapy apparatus is one type or form of respiratory apparatus to which the predictive alarm may be applied, and is explained by way of example only.

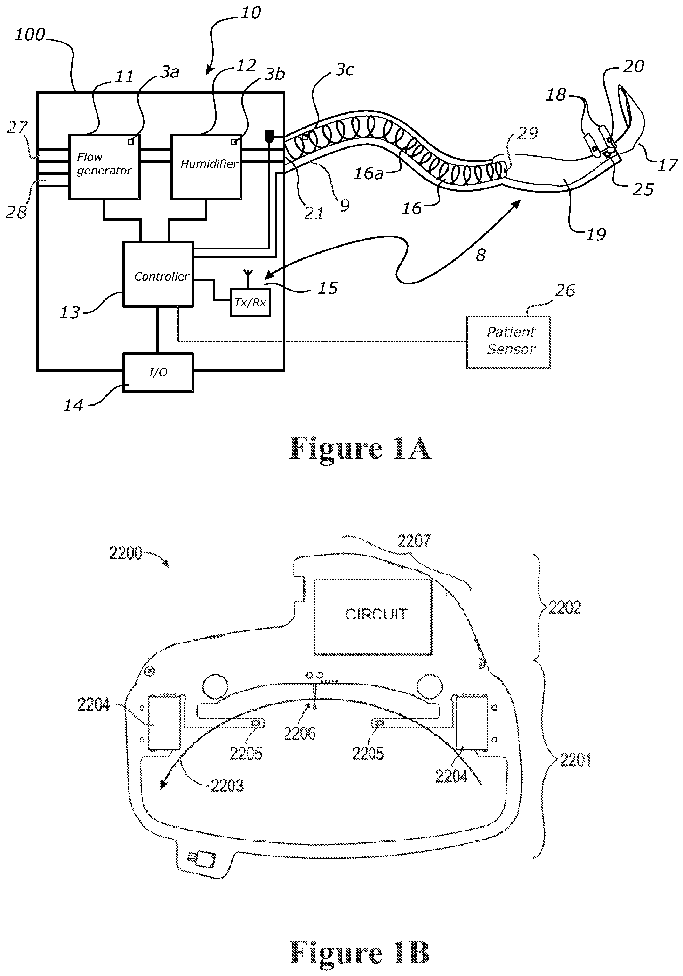

A flow therapy apparatus 10 is shown in Figure 1A. The apparatus 10 can comprise a main housing 100 that contains a flow generator 11 in the form of a motor/impeller arrangement (for example, a blower), an optional humidifier 12, a controller 13, and a user interface 14 (comprising, for example, a display and input device(s) such as button(s), a touch screen, or the like). The controller 13 can be configured or programmed to control the operation of the apparatus. For example, the controller can control components of the apparatus, including but not limited to: operating the flow generator 11 to create a flow of gas (gases flow) for delivery to a patient, operating the humidifier 12 (if present) to humidify and/or heat the generated gases flow, control a flow of oxygen into the flow generator blower, receiving user input from the user interface 14 for reconfiguration and/or user-defined operation of the apparatus 10, and outputting information (for example on the display) to the user. The user can be a hospital patient, homecare patient or user, healthcare professional (e.g. nurse, clinician, doctor), or anyone else interested in using or operating the apparatus. As used herein, unless the context suggests otherwise, a "gases flow" can refer to any flow of gases that may be used in the breathing assistance or respiratory device, such as a flow of ambient air, a flow comprising substantially 100% oxygen, a flow comprising some combination of ambient air and oxygen, and/or the like.

As explained above, the humidifier of the apparatus is preferable but not essential. Adding humidity to the gases stream improves comfort to the therapy, and improves patient compliance of the therapy and prevents or minimises drying out of the patient's airways. For high flow therapy, a humidifier can be particularly advantageous as high flow without humidity can dry the airways and reduce function of the lungs e.g. reducing mucociliary transport action. Humidity helps to improve comfort by keeping lungs warm and moist and improves/maintains healthy mucociliary transport action. Humidity may also help to maintain physiological stability in compromised airways and may help with compliance to the therapy.

A patient breathing conduit 16 is coupled at one end to a gases flow outlet 21 in the housing 100 of the flow therapy apparatus 10. By way of example, the patient breathing conduit may be provided with a conduit connector at one end that couples to an electrical/pneumatic connector on the housing 100 of the flow therapy apparatus. The conduit connector of the patient breathing tube has a pneumatic coupling to facilitate gases flow from the apparatus to the conduit, and an electrical connector. The electrical connector couples a heater wire of the breathing conduit to the electrical circuitry and/or controller of the flow therapy apparatus. The patient breathing conduit 16 is coupled at another end to a patient interface 17 such as a non-sealed nasal cannula with a manifold 19 and nasal prongs 18. Additionally, or alternatively, the patient breathing conduit 16 can be coupled to a face mask, a nasal mask, a nasal pillows mask, an endotracheal tube, a tracheostomy interface, and/or the like. The gases flow that is generated by the flow therapy apparatus 10 may be humidified, and delivered to the patient via the patient conduit 16 through the cannula 17. The patient conduit 16 can have a heater wire 16a to heat gases flow passing through to the patient. The heater wire 16a can be under the control of the controller 13. The patient conduit 16 and/or patient interface 17 can be considered part of the flow therapy apparatus 10, or alternatively peripheral to it. The flow therapy apparatus 10, breathing conduit 16, and patient interface 17 together can form a flow therapy system.

The controller 13 can control the flow generator 11 to generate a gases flow at the desired flow rate. By way of example, in one configuration, the controller is configured to control to the desired flow rate by controlling the motor speed of the blower. In this configuration, the controller receives a target or set flow rate. The controller then calculates a set motor speed that corresponds to the target flow rate. In one configuration, the set motor speed may be calculated based on a target or set flow rate and a model or equation/function or a look-up table. By way of example, the model or equation/function or look-up table may relate flow rate generate to set motor speed. In another configuration, the set motor speed may be calculated based on a function, model, or equation input, and one or more sensor inputs (e.g. motor speed sensor and/or flow rate sensor and/or other sensors). The controller 13 can also control a supplemental oxygen inlet to allow for delivery of supplemental oxygen, the humidifier 12 (if present) can humidify the gases flow and/or heat the gases flow to an appropriate level, and/or the like. In this configuration, the humidifier 12 comprises a water or humidification chamber component through which the gases flows, and a heater plate or heater component that is configured to heat the water in the chamber. The gases flow is directed out through the patient conduit 16 and cannula 17 to the patient. The controller 13 can also control a heating element (for example a heater plate of a water or humidification chamber through which the gases flow) in the humidifier 12 and/or the heating element 16a in the patient conduit 16 to heat the gas to a desired temperature for a desired level of therapy and/or level of comfort for the patient. The controller 13 can be programmed with or can determine a suitable target temperature of the gases flow.

The oxygen inlet port 28 can include a valve through which a pressurized gas may enter the flow generator or blower. The valve can control a flow of oxygen into the flow generator (e.g. blower). The valve can be any type of valve, including a proportional valve or a binary valve. In one configuration the valve is preferably a proportional-type valve to allow the apparatus to control to various concentrations of oxygen. The oxygen supply through the valve may be a high pressure oxygen source. Optionally the apparatus may include an additional oxygen inlet to allow low pressure oxygen to be introduced into the respiratory apparatus. The high-pressure and/or low-pressure oxygen is mixed with ambient air drawn into the apparatus through an ambient air inlet or inlets prior to the oxygen air moisture being humidified. The source of oxygen can be an oxygen tank or a hospital oxygen supply. Medical grade oxygen is typically between 95% and 100% purity. Oxygen sources of lower purity can also be used. In other configurations, the atmospheric air may be augmented with any other suitable supplemental gas such as, but not limited to, Nitrogen, Heliox, Nitrous oxide. Again, a supplemental gas inlet port may have a controllable valve to control the flow rate or supply of the supplemental gas to thereby allow control over the concentration and/or composition of the gases with within the gases stream or flow of gases delivered to the end user or patient.

The flow therapy apparatus 10 can measure and control the oxygen content of the gas being delivered to the patient, and therefore the oxygen content of the gas inspired by the patient. During high flow therapy, the high flow rate of gas delivered is typically close to, at, or above the peak inspiratory demand of the patient. This means that the volume of gas delivered by the device to the patient during inspiration meets, or is in excess of, the volume of gas inspired by the patient during inspiration. High flow therapy therefore helps to prevent entrainment of ambient air when the patient breathes in, as well as flushing the patient's airways of expired gas. So long as the flow rate of delivered gas meets or exceeds peak inspiratory demand of the patient, entrainment of ambient air is prevented, and the gas delivered by the device is substantially the same as the gas the patient breathes in. As such, the oxygen concentration measured in the device, fraction of delivered oxygen, (FdO2) would be substantially the same as the oxygen concentration the user is breathing, fraction of inspired oxygen (FiO2), and as such the terms may can be seen as equivalent.

Operation sensors 3a, 3b, 3c, such as flow, temperature, humidity, and/or pressure sensors can be placed in various locations in the flow therapy apparatus 10. Additional sensors (for example, sensors 20, 25) may be placed in various locations on the patient conduit 16 and/or cannula 17 (for example, there may be a temperature sensor 29 at or near the end of the inspiratory tube). Output from the sensors can be received by the controller 13, to assist the controller in operating the flow therapy apparatus 10 in a manner that provides suitable therapy. In some configurations, providing suitable therapy includes meeting (e.g. delivery gases at a flow rate close to, at, or above) a patient's peak inspiratory demand. The apparatus 10 may have a transmitter and/or receiver 15 or communication module to enable the controller 13 to receive signals 8 from the sensors and/or to control the various components of the flow therapy apparatus 10, including but not limited to the flow generator 11, humidifier 12, and heater wire 16a, or accessories or peripherals associated with the flow therapy apparatus 10. Additionally, or alternatively, the transmitter and/or receiver 15 or communication module may deliver data to a remote server or enable remote control of the apparatus 10, or may transmit data to any other remote device or system such as, but not limited to, an external monitoring device or system. In some configurations, the transmitter and/or receiver 15 may be or may comprise any one or more of the following: Modem, Wifi module, Bluetooth module, infra-red module, and/or NFC module. In one example configuration, the respiratory apparatus comprises at least one or more of a Modem, Wifi module, Bluetooth module, infra-red module, NFC module, and/or any other wireless communication module to allow communication of data (e.g. over a wireless communication link or wireless data link) such as, but not limited to, usage data, compliance data and/or apparatus-related data. By way of example, apparatus-related data may include data such as, but not limited to, alarm data relating to alarms that have triggered, sensor data, or other data that can be used to perform diagnostics on the apparatus. This apparatus-related data can be transmitted or sent to an external server or processing system, where the server or system can process the data to identify faults or perform other diagnostics based on the apparatus-related data. This processing allows for repair and/or maintenance of the apparatus based on the output data from the diagnostic analysis.

Oxygen may be measured by placing one or more gas composition sensors (such as an ultrasonic transducer system, also referred to as an ultrasonic sensor system) after the oxygen and ambient air have finished mixing. The measurement can be taken within the device, the delivery conduit, the patient interface, or at any other suitable location. By way of example, a gas composition sensor (e.g. an oxygen concentration sensor) may be positioned between the blower and humidifier. The blower mixes the incoming oxygen supply with ambient air to generate or form an oxygen-augmented gases stream. The oxygen concentration sensor may be positioned within the bulk gases flow path (i.e. main gases flow path from the inlets, through the blower, humidifier, and to the outlet) of the apparatus, or the sensor may be located adjacent or separate to the bulk gases flow path, such as in a sampling passage or path branching off or otherwise pneumatically connected or in fluid communication with the bulk gases flow path.

The flow therapy apparatus 10 can include a patient sensor 26, such as a pulse oximeter or a patient monitoring system, to measure one or more physiological parameters of the patient, such as a patient's blood oxygen saturation (SpO2), heart rate, respiratory rate, perfusion index, and provide a measure of signal quality. The sensor 26 can communicate with the controller 13 through a wired connection or by communication through a wireless transmitter on the sensor 26. The sensor 26 may be a disposable adhesive sensor designed to be connected to a patient's finger. The sensor 26 may be a non-disposable sensor. Sensors are available that are designed for different age groups and to be connected to different locations on the patient, which can be used with the flow therapy apparatus. The pulse oximeter would be attached to the user, typically at their finger, although other places such as an earlobe are also an option. The pulse oximeter would be connected to a processor in the device and would constantly provide signals indicative of the patient's blood oxygen saturation. The patient sensor 26 can be a hot swappable device, which can be attached or interchanged during operation of the flow therapy apparatus 10. For example, the patient sensor 26 may connect to the flow therapy apparatus 10 using a USB interface or using wireless communication protocols (such as, for example, near field communication (NFC), WiFi or Bluetooth®). When the patient sensor 26 is disconnected during operation, the flow therapy apparatus 10 may continue to operate in its previous state of operation for a defined time period. After the defined time period, the flow therapy apparatus 10 may trigger an alarm, transition from automatic mode to manual mode, and/or exit control mode (e.g., automatic mode or manual mode) entirely. The patient sensor 26 may be a bedside monitoring system or other patient monitoring system that communicates with the flow therapy apparatus 10 through a physical or wireless interface.

The flow therapy apparatus 10 may comprise a high flow therapy apparatus. High flow therapy as discussed herein, unless the context suggests otherwise, is intended to be given its typical ordinary meaning as understood by a person of skill in the art, which generally refers to a respiratory assistance system delivering a targeted flow of humidified respiratory gases via an intentionally unsealed patient interface with flow rates generally intended to meet or exceed inspiratory flow of a patient. Typical patient interfaces are unsealed interfaces and include, but are not limited to, nasal interfaces (e.g. cannula), tracheal interfaces, or oral interfaces. Typical flow rates for adults often range from, but are not limited to, about fifteen liters per minute (LPM) to about seventy liters per minute or greater. Typical flow rates for pediatric patients (such as neonates, infants and children) often range from, but are not limited to, about one liter per minute per kilogram of patient weight to about three liters per minute per kilogram of patient weight or greater. High flow therapy can also optionally include gas mixture compositions including supplemental oxygen and/or administration of therapeutic medicaments.

By way of example only, high flow therapy is often referred to as nasal high flow (NHF), humidified high flow nasal cannula (HHFNC), high flow nasal oxygen (HFNO), high flow therapy (HFT), or tracheal high flow (THF), among other common names. By way of example only, the flow rates used to achieve "high flow" may be any of the flow rates listed below. For example, in some configurations, for an adult patient 'high flow therapy' may refer to the delivery of gases to a patient at a flow rate of greater than or equal to about 10 litres per minute (10 LPM), such as between about 10 LPM and about 100 LPM, or between about 15 LPM and about 95 LPM, or between about 20 LPM and about 90 LPM, or between about 25 LPM and about 75 LPM, or between about 25 LPM and about 85 LPM, or between about 30 LPM and about 80 LPM, or between about 35 LPM and about 75 LPM, or between about 40 LPM and about 70 LPM, or between about 45 LPM and about 65 LPM, or between about 50 LPM and about 60 LPM. In some configurations, for a neonatal, infant, or child patient 'high flow therapy' may refer to the delivery of gases to a patient at a flow rate of greater than 1 LPM, such as between about 1 LPM and about 25 LPM, or between about 2 LPM and about 25 LPM, or between about 2 LPM and about 5 LPM, or between about 5 LPM and about 25 LPM, or between about 5 LPM and about 10 LPM, or between about 10 LPM and about 25 LPM, or between about 10 LPM and about 20 LPM, or between about 10 LPM and about 15 LPM, or between about 20 LPM and about 25 LPM. A high flow therapy apparatus with an adult patient, a neonatal, infant, or child patient, may deliver gases to the patient at a flow rate of between about 1 LPM and about 100 LPM, or at a flow rate in any of the sub-ranges outlined above.

The flow therapy apparatus 10 can deliver any concentration of oxygen (e.g., FdO2), up to 100%, at any flowrate between about 1 LPM and about 100 LPM. In some configurations, any of the flowrates can be in combination with oxygen concentrations (FdO2s) of about 20%-30%, 21%-30%, 21%-40%, 30%-40%, 40%-50%, 50%-60%, 60%-70%, 70%-80%, 80%-90%, and 90%-100%. In some combinations, the flow rate can be between about 25 LPM and 75 LPM in combination with an oxygen concentration (FdO2) of about 20%-30%, 21%-30%, 21%-40%, 30%-40%, 40%-50%, 50%-60%, 60%-70%, 70%-80%, 80%-90%, and 90%-100%. In some configurations, the flow therapy apparatus 10 may include safety thresholds when operating in manual mode that prevent a user from delivering to much oxygen to the patient.

High flow therapy may be administered to the nares of a user and/or orally, or via a tracheostomy interface. High flow therapy may deliver gases to a user at a flow rate at or exceeding the intended user's peak inspiratory flow requirements. The high flow therapy may generate a flushing effect in the nasopharynx such that the anatomical dead space of the upper airways is flushed by the high incoming gases flow. This can create a reservoir of fresh gas available for each and every breath, while minimizing re-breathing of nitrogen and carbon dioxide. Meeting inspiratory demand and flushing the airways is additionally important when trying to control the patient's FdO2. High flow therapy can be delivered with a non-sealing patient interface such as, for example, a nasal cannula. The nasal cannula may be configured to deliver breathing gases to the nares of a user at a flow rate exceeding the intended user's peak inspiratory flow requirements.

The phrases "non-sealing patient interface" or "unsealed interface" as used herein, unless the context suggests otherwise, can refer to an interface providing a pneumatic link between an airway of a patient and a gases flow source (such as from flow generator 11) that does not completely occlude the airway of the patient. In one configuration, the non-sealed pneumatic link can comprise an occlusion of less than about 95% of the airway of the patient. In another configuration, the non-sealed pneumatic link can comprise an occlusion of less than about 90% of the airway of the patient. In another configuration, the non-sealed pneumatic link can comprise an occlusion of between about 40% and about 80% of the airway of the patient. The airway can include one or more of a nare or mouth of the patient. For a nasal cannula the airway is through the nares.

The flow generator or blower 11 can include an ambient air inlet port 27 to entrain ambient room air into the blower. The flow therapy apparatus 10 may also include an oxygen inlet port 28 leading to a valve through which a pressurized gas may enter the flow generator or blower 11. The valve can control a flow of oxygen into the flow generator blower 11. The valve can be any type of valve, including a proportional valve or a binary valve.

The blower can operate at a motor speed of greater than about 1,000 RPM and less than about 30,000 RPM, greater than about 2,000 RPM and less than about 21,000 RPM, greater than about 4,000 RPM and less than about 19,000 RPM or between any of the foregoing values. Operation of the blower can mix the gases entering the blower through the inlet ports. Using the blower as the mixer can decrease the pressure drop that would otherwise occur in a system with a separate mixer, such as a static mixer comprising baffles, because mixing requires energy. Having a static mixer can also increase the volume of the gas flow path between the valve and the gases composition sensor, which can further increase the delay between when the valve current is changed and when a corresponding change in oxygen concentration is measured.

Based on user inputs and the therapy supplied by the specific device, the controller can determine a target output parameter for the blower. The controller can receive measurements of the target output parameter, and based on the difference between determined flow rate and the measured flow rate, the controller can adjust the speed of the blower.

The target output parameter may be flow rate. The target flow rate may be a constant value, (e.g., nasal high flow). The target flow rate may be a value that fluctuates. In some configurations, the controller can control a blower motor speed based on a target flow rate, and additionally increase or decrease the motor speed based on a patient's breathing cycle. The target flow rate does not necessarily change, but the controller may be configured to control the motor speed to fluctuate in order add oscillations to the instantaneous flow rate such that the flow rate is synchronized with the patient's breathing. Such a system is described in International Application PCT Publication No. WO/2017/200394, titled "Flow Path Sensing for Flow Therapy Apparatus".

The target output parameter may alternatively be pressure. The target pressure may be a constant value, for example Continuous Positive Airway Pressure (CPAP). Alternatively, the target pressure may a value that fluctuates, potentially in time with the breath, for example Bi-Level pressure therapy. In both of these scenarios, the total flow rate is unlikely to be constant.

With additional reference to Figure 1B, a sensing circuit board 2200 is shown that can be implemented in the flow therapy apparatus 10. The sensing circuit board 2200 can be positioned in a sensor chamber such that the sensing circuit board 2200 is at least partially immersed in the flow of gases. The flow of gases may exit the blower 11 through a conduit and enter a flow path in the sensor chamber. At least some of the sensors on the sensing circuit board 2200 can be positioned within the flow of gases to measure gas properties within the flow. After passing through the flow path in the sensor chamber, the gases can exit to the humidifier 12 described above.

The sensing circuit board 2200 can be a printed sensing circuit board (PCB). Alternatively, the circuit on the board 2200 can be built with electrical wires connecting the electronic components instead of being printed on a circuit board. At least a portion of the sensing circuit board 2200 can be mounted outside of a flow of gases. The flow of gases can be generated by the flow generator 11 described above. The sensing circuit board 2200 can comprise ultrasonic transducers 2204. The sensing circuit board 2200 can comprise one or more of thermistors 2205. The thermistors 2205 can be configured to measure a temperature of the gases flow. The sensing circuit board 2200 can comprise a thermistor flow rate sensor 2206. The sensing circuit board 2200 can comprise other types of sensors, such as humidity sensors including humidity only sensors to be used with a separate temperature sensor and combined humidity and temperature sensors, sensors for measuring barometric pressure, sensors for measuring differential pressure, and/or sensors for measuring gauge pressure. The thermistor flow rate sensor 2206 can comprise hot wire anemometer, such as a platinum wire, and/or a thermistor, such as a negative temperature coefficient (NTC) or positive temperature coefficient (PTC) thermistor. Other non-limiting examples of the heated temperature sensing element include glass or epoxy-encapsulated or non-encapsulated thermistors. The thermistor flow rate sensor 2206 can be configured to measure flow rate of the gases by being supplied with a constant power, or be maintained at a constant sensor temperature or a constant temperature difference between the sensor and the flow of gases. In one example configuration of the respiratory apparatus, the sensing circuit board is positioned or located between the blower and humidifier (i.e. in a location near, partially within, or adjacent to the gases flow path extending between the blower and humidifier). This is advantageous because gases parameters or characteristics of the gases in the gases stream are sensed by the one or more sensors of the sensing circuit board after the inlet gases (e.g. ambient air and oxygen and/or other supplementary gases) have been mixed. Also, sensing gases parameters or characteristics prior to the humidifier ensures the humidified gases do not contact or interact with the sensors, and this reduces the chances of condensate of or within the sensors. Also, sensing of the gases stream prior to humidification provides more accurate and/or reliable sensor measurements.

The sensing circuit board 2200 can comprise a first portion 2201 and a second portion 2202. The first portion 2201 can be positioned to be within the flow path of the gases, whereas the second portion 2202 can be positioned to be outside the flow path of the gases. The direction of the flow of gases is indicated in Figure 1B by the arrow 2203. The direction of the flow of gases can be a straight line, or curved as shown in Figure1B.

Positioning the one or more of thermistors 2205 and/or the thermistor flow rate sensor 2206 downstream of the combined blower and mixer can take into account heat supplied to the gases flow from the blower. Also, immersing the temperature-based flow rate sensors in the flow path can increase the accuracy of measurements because the sensors being immersed in the flow are more likely to be subject to the same conditions, such as temperature, as the gases flow and therefore provide a better representation of the gases characteristics.

The sensing circuit board 2200 can comprise ultrasonic transducers, transceivers, or sensors of the sensing circuit board to measure gases properties of the gases flow, such as gas composition or concentration of one or more gases within the gases stream. Any suitable transducer, transceiver, or sensor may be mounted to the sensing circuit board 2200 as will be appreciated. In this configuration, the sensing circuit board includes an ultrasonic transducer system (also referred to as an ultrasonic sensor system) that employs ultrasonic or acoustic waves for determining gas concentrations. As will be appreciated, the ultrasonic transducers can be used in a gas composition sensing system for sensing the speed of sound through the gases stream by the transmission and reception of ultrasonic beams or pulses.

The ultrasonic transducer system may determine the relative gas concentrations of two or more gases in the gases flow. The ultrasonic transducer system may be configured to measure the oxygen fraction in the bulk gases stream flow, which consists of atmospheric air augmented with supplemental oxygen, which is essentially a binary gas mixture of nitrogen (N2) and oxygen (O2). It will also be appreciated that the ultrasonic transducer system may be configured to measure the gas concentrations of other augmentation gases that have blended with atmospheric air in the gases stream, including nitrogen (N2) and carbon dioxide (CO2). The ultrasonic transducers can determine the gas concentration of gases in the gases flow at a relatively high frequency. For example, the ultrasonic transducers can output a measured FdO2 value at a maximum sample rate of the sensors or at a lower frequency than the maximum sample rate, such as between about 1 Hz and 200 Hz, about 1 Hz and 100 Hz, about 1 Hz and 50 Hz, and about 1 Hz and 25 Hz.

In some configurations, the flow therapy apparatus may also be provided with a humidity sensor that is located in the flow path and which is configured to generate a humidity signal indicative of the humidity of the gases stream flowing through the sensor assembly. In such embodiments, the gas composition may be determined by the sensed speed of sound, and the sensed temperature and/or sensed humidity of the gases stream. The humidity sensor may be a relative humidity sensor or an absolute humidity sensor. In some embodiments, the gas composition may be determined based on the sensed speed of sound and the sensed humidity, without the need for a temperature sensor.

The ultrasonic transducer system may be used to measure respective ratios of any two known gases in a gas composition. The ultrasonic transducer system can determine the relative gas concentration in a mixture of air blended with supplementary oxygen, which is substantially equivalent to a nitrogen/oxygen mixture. In such a binary gas mixture, by monitoring the speed of sound and taking the temperature into account, the mean molecular weight of the gas can be determined, and thus, the relative concentrations of the two gases may be determined. From this ratio, the oxygen fraction or nitrogen fraction of the gases stream may be extracted.

In some configurations, the flow therapy apparatus can measure the total flow rate of gases using a flow bead, as is described in International Application Publication No. WO/2018/052320, titled "Thermistor Flow Sensor Having Multiple Temperature Points". The flow bead can have the advantage of providing a more accurate measure of the flow rate, however it can be slow to react to sudden changes in the flow (such as high frequency oscillations). The ultrasonic transducers can measure sudden changes in the flow, however the overall measurement can be less accurate. In some configurations, the controller can generate a final measurement of the total flow rate by combining inputs from a flow bead and ultrasonic transducers, thereby allowing for a measure of the flow rate that is both accurate and able to detect sudden changes in the flow.

Some examples of flow therapy apparatuses are disclosed in International Application Publication No. WO/2017/095241, titled "Flow Path Sensing for Flow Therapy Apparatus", and International Application Publication No. WO/2016/207838, titled "Breathing Assistance Apparatus".

### 3. <u>Overview of predictive over-temperature alarm and control method</u>

Some example embodiments of the predictive over-temperature alarm will now be explained more specifically in the context of a respiratory apparatus in the form of a flow therapy apparatus of the type described above, by way of example only. This flow therapy apparatus provides high flow therapy and thereby provides respiratory support. As previously discussed, the alarm may also be adapted to other types of respiratory apparatus, including those capable of operating in a plurality of modes and/or capable of delivering a plurality of therapies. In one example, the alarm may be applied to a respiratory apparatus that is capable of delivering both high flow therapy and pressure therapy, depending on the mode of operation selected, and the alarm may function with either therapy or mode or operation. In one configuration, the controller of the apparatus may be configured with different variants and/or versions of the alarm algorithm suitable for the different respective modes of operation or therapies offered by the apparatus, and can automatically apply or implement the version of the alarm algorithm suitable to the current or selected mode of operation or therapy to be delivered by the apparatus.

The predictive over-temperature alarm is an algorithm, method or process carried out or executed by the main controller or a sub-controller of the respiratory apparatus during operation of the apparatus. The predictive over-temperature alarm can be configured to predict whether at least one of the input settings or parameters applied to the respiratory apparatus for a therapy session can be applied or maintained without the steady-state temperature of the device exceeding a predetermined high-temperature condition, which may be a maximum temperature or threshold parameter for example. As will be appreciated, a therapy session is typically a session or time period of operation of the apparatus in which a user receives a flow of gases (e.g. typically humidified gases) from the apparatus having one or more particular flow characteristics.

In this example, the respiratory apparatus is a flow therapy apparatus in which the primary input setting or parameter is a flow rate setting which determines the flow rate of the gases flow delivered to the user during the therapy session. In this example configuration, the predictive over-temperature alarm is configured to predict whether the set flow rate for a desired, current, or new therapy session can be achieved, and maintained, without the device's steady-state temperature exceeding the predetermined maximum temperature or threshold. More specifically the set flow rate corresponds to a motor speed of the blower, and the controller determines if the required motor speed (for the set flow rate) is going to cause the steady-state temperature to increase above a preset high-temperature condition-threshold.

In this example configuration, in addition to the predictive over-temperature alarm, the apparatus may have an internal-device high-temperature alarm. If the internal temperature of the apparatus exceeds a threshold as measured by one or more internal temperature sensors, then a high temperature alarm is triggered or raised. The temperature sensor or sensors may comprise a temperature sensor on the sensing circuit board or may be a temperature sensor located elsewhere in the housing of the apparatus such as, but not limited to, a temperature sensor in the outlet of the apparatus or a temperature sensor adjacent or near the display screen of the user interface. If the internal-device high-temperature alarm is triggered based on the sensed temperature, this indicates that the internal apparatus temperature is risky as it may cause damage to other sensors or electronics or other components.

The controller may be configured to undertake one or more actions or alarm responses automatically if the internal-device high-temperature alarm is triggered. Various examples of the one or more actions or alarm responses are provided below. Any combination of one or more of the following actions or alarm responses may be triggered or undertaken by the controller if the internal-device high-temperature alarm is triggered:

- the controller may automatically reduce the motor speed of the blower (and hence the delivered or generated flow rate) to a level that will reduce internal apparatus temperature.
- the controller may indicate or notify the user to change the size of the patient interface. For example, if the interface is a cannula or tracheal interface, the controller may prompt or notify the user to change to a larger cannula or larger tracheal interface, because the size of current cannula or tracheal interface is creating too much resistance to flow and hence causing the motor speed to be too high for the set flow rate, resulting in the over-temperature condition.
- the controller may be configured to clear or reset the internal-device high-temperature alarm when the sensed temperature of the apparatus cools below a threshold (which may be the same or different to the alarm threshold temperature).
- the controller may be configured to reduce the heater plate temperature set point of the humidifier. This alarm response can result in a reduced humidity output of the gases flow, but may also lower heat energy output from the heater plate into the apparatus generally, and therefore may assist in reducing heat energy and resulting temperature within the apparatus.
- the controller may present a message or notification to the user via the user interface display to check for a blockage within the gases flow circuit, which may be contributing or causing the over-temperature condition.
- the controller may cause the blower to be driven in reverse to extract hot gases and expel them out of the apparatus to cool the apparatus.
- the controller may present a general message or notification to the user on a display of the apparatus indicating that the internal-device high-temperature alarm has triggered.
- the controller may shut-down the apparatus and halt current operation or therapy.

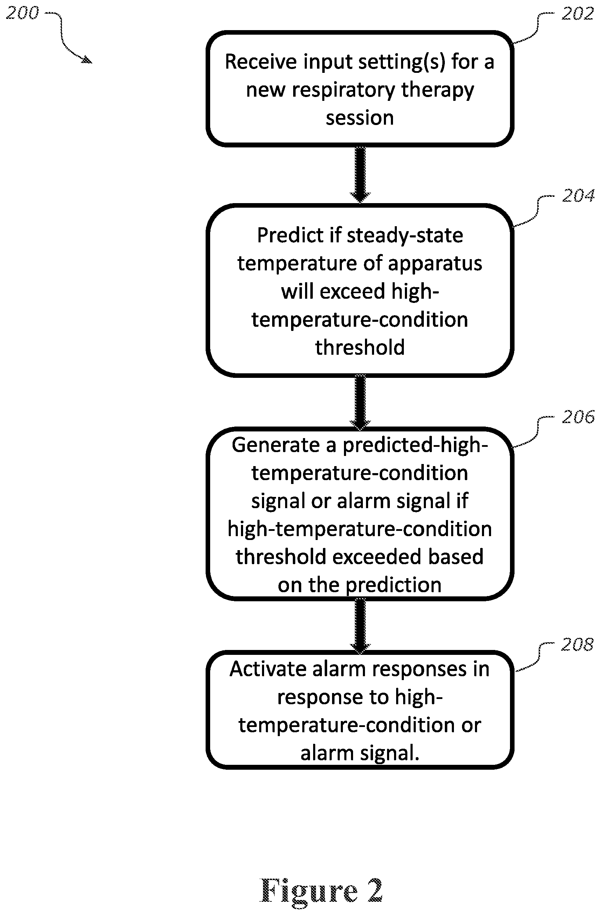

Referring to Figure 2, a summary of the predictive over-temperature alarm process flow or predictive alarm method 200 implemented by a controller of the respiratory apparatus will be described. The predictive alarm method 200 commences by receiving input data representing one or more input settings or parameters relating to the desired or new therapy session to be commenced as shown at 202. By way of example, the input setting may be a set flow rate indicative of the desired flow rate of the gases flow to be delivered to the user of the apparatus during the therapy session. The predictive alarm method 200 proceeds to then predict if the steady-state temperature of the respiratory apparatus will exceed the high-temperature condition threshold based on the input setting(s) and/or one or more associated operational parameters related to the input setting(s), as shown at 204. By way of example, the controller can be configured to predict if the steady-state temperature of the respiratory apparatus will exceed the high-temperature condition threshold based on the set flow rate input and/or the motor speed of the blower required to generate the set flow rate. If the predicted steady state temperature exceeds a predetermined high-temperature condition threshold, a predicted high-temperature alarm or alarm signal is generated or triggered based on the prediction, as shown at 206. Optionally, in response to a generated alarm or alarm signal, the controller can be configured to initiate or activate one or more alarm responses, as shown at 208.

The predictive alarm method 200 at step 204 involves predicting a steady-state temperature of or associated with the apparatus based at least partly on a predetermined or dynamic heat model. The heat model represents one or more heat sources of or associated with the apparatus. More specifically, the heat model may represent the heat generated by one or more components (heat sources) of the apparatus in terms of the heat energy generated by the components that contributes or causes an increase to the steady-state temperature of the apparatus that is being determined by the predictive over-temperature alarm. In one configuration, the heat model at least represents the heat generated by the blower of the flow therapy apparatus as a function of motor speed and flow rate. In another configuration, the heat model further represents the heat generated by one or more non-blower heat sources of or associated with the apparatus that will contribute to the steady-state temperature of the apparatus. By way of example only, the one or more non-blower heat sources can include, but it not limited to, any one or more of the following non-blower components or environmental variables of, connected to, or associated with the apparatus: a battery or power source, a heater plate of a humidifier, a heater wire of a patient interface conduit, electronic circuitry or components, a Printed Circuit Board (PCB), a graphical user interface, a display screen, and/or ambient conditions (such as, but not limited to, the ambient temperature and/or ambient humidity) of environment surrounding the apparatus. As will be appreciated, the heat model may be configured to model heat sources in metrics of temperature (such as degrees Celsius or Fahrenheit or similar) and/or metrics of heat energy (such as, but not limited to, Joules or other energy SI units).

In a first form, the heat model may be at least partly on a theoretical heat model of the apparatus. For example, the theoretical heat model may be a computer or mathematical or scientific model or calculation of how one or more heat sources in or associated with the apparatus may contribute to the steady-state temperature of the apparatus. In a second form, the heat model can be based at least partly on an experimental heat model of the apparatus based on experimental heat data associated with operation of the apparatus, for example via experimental testing. The experimental heat data may be based on recorded or stored temperature data that is measured or sensed over one or more operating ranges, conditions, and/or therapy sessions of the apparatus in use or experimental testing for example. In a third form, the heat model can be based at least partly on one or more predetermined or configurable safety values. For example, the heat model may take into account one or more heat sources like ambient temperature or heat generated by heater elements or such components, and may allocate a safety heat value to those parameters or components that represents a likely or realistic upper limit to the maximum heat they each might contribute to the steady-state temperature of the apparatus in operation. Any of the above heat models may be stored or saved in memory of the controller of the apparatus. As will be appreciated, the heat model may be represented as a mathematical function, look-up table, or any other suitable computable format.

Predicting a steady-state temperature of or associated with the apparatus at step 204 based on the heat model involves the controlling determining the motor speed of the blower that is required to generate the input set flow rate target for the therapy session, and using that determined motor speed as an input or variable into the heat model for generating the predicted steady-state temperature of the apparatus. In some apparatus configurations, the controller is configured to determine the motor speed required to generate the set flow rate based on or by extracting or estimating that data a stored known relationship or model or function of motor speed and flow rate.

In other configurations, the controller is configured to determine the required motor speed to generate the input set flow rate by increasing or changing the motor speed of the blower until the set flow rate is generated, such that the required motor speed is determined in real-time or dynamically for the particular apparatus and current operation variables. This real-time or dynamic determination of the required motor speed enables the controller to take into account the current apparatus configuration characteristics and/or parameters for the individual user, such as the type and configuration of the patient interface being used, type and characteristics of the patient interface conduit being used, and/or any other parameters or characteristics of the apparatus in operation that may have an impact on the relationship between motor speed and flow rate generated.

The predictive alarm method 200 is implemented by the controller whenever a new set flow rate input is received by a user, which typically occurs when a new therapy session is being commenced for a user or if the set flow rate is changed or modified during a therapy session. The flow therapy apparatus may start a new therapy session from a start-up or stand-by mode in which the blower is stopped or idling at a non-therapy speed, or may be switched to a new desired therapy session or a modified or new set flow rate while the apparatus is already operating in a current therapy session. In either scenario, the predictive alarm method 200 is implemented by the controller in response to receiving the new or updated set flow rate input associated with the desired therapy session, and determining whether the predicted steady-state temperature of the apparatus will exceed the high-temperature-condition threshold based on those new or updated input setting(s) and/or parameters associated with the new desired therapy session.

The predictive alarm method 200 may be configured to predict or determine a future steady-state temperature of interest of, within, or associated with the apparatus. The type or nature of the steady-state temperature of interest may depend on the required purposes or objectives of the alarm. The predictive alarm is configured to predict a steady-state temperature that represents a sustained and/or stable temperature parameter, as opposed to a transient temperature parameter. For example, the predictive alarm is directed at predicting whether a temperature aspect of the apparatus will be maintained in an undesirable or unsafe over-temperature zone for an undesirable and/or unsafe period, as opposed to predicting whether a temperature aspect might briefly spike into an unsafe zone in future. The predictive alarm is primarily directed at alarming if it is predicted that the apparatus, a region within the apparatus, a component of the apparatus, or a parameter relating to the apparatus or its operation is going to experience a sustained period of high heat (e.g. an overheat state or an over-temperature condition) that is detrimental in some way to the apparatus and/or user, depending on the particular steady-state temperature of interest.

By way of example, in one configuration, the predictive alarm may be configured such that the steady-state temperature predicted by the alarm method represents a predicted temperature parameter of the apparatus after it has been operating at the set flow rate for at least a predetermined minimum time period. In other words, in one configuration, the predicted steady-state temperature determined by the alarm method represents a likely settled or long-term averaged temperature of the apparatus. The time period may be such that the steady-state temperature represents a sustained and/or stable temperature parameter, for example once the apparatus has settled into a relatively stable operating condition, as opposed to being in a 'start-up' condition where at least some components may not be up to their long-term operating temperature and/or condition for the therapy session.

As discussed above, the predictive alarm can be configured such that the steady-state temperature of interest for the alarm is any desired temperature parameter of, within, or associated with the respiratory apparatus.

In one configuration, the steady-state temperature for the predictive alarm can be configured to represent the predicted temperature of the flow of gases in the apparatus at a specified region or location within a gases flow path of the apparatus. For example, in this configuration, the steady-state temperature may represent the predicted temperature of the flow of gases after the humidifier and/or at or near the outlet of the apparatus which connects to the patient interface conduit, or the predicted temperature of the flow of gases in the region of one or more sensors and/or a sensing circuit or PCB of the apparatus.

In another configuration, the steady-state temperature for the predictive alarm can be configured to represent the predicted temperature within a specific location or region of a main housing of the apparatus or at or in the region of a specific component or componentry of the apparatus. By way of example only, the apparatus may be configured such that the steady-state temperature may be configured to represent the predicted temperature at any one of the following selected locations or regions:

- within a central region or zone of the housing of the apparatus,
- a location or region near the blower of the apparatus,
- a location or region near electronic circuitry or a PCB of the apparatus,
- a location or region near a display screen or user interface componentry of the apparatus,
- a location or region near the humidifier or components of the humidifier of the apparatus, or
- a location or region near specific componentry, such as sensors or sensing circuitry. By way of example, the sensors or sensing circuity may comprise any one or more of the following: flow sensors, temperature sensors, pressure sensors, humidity sensors, ultrasonic sensors or the like, which may be temperature sensitive in their accuracy or sensitivity or which might only reliably operate within specific operating temperature ranges.

In another configuration, the steady-state temperature for the predictive alarm can be configured to represent a predicted generic overall temperature parameter associated with the apparatus based on a heat model of the apparatus and/or one or more heat sources of interest of or associated with the apparatus. For example, in this configuration, the steady-state temperature may represent a theoretical or overall temperature parameter that is not specific to a specific location, region, or component of the apparatus, but is a general temperature parameter representing the total temperature or heat in or associated with the apparatus or device.

Generally, the purpose of the predictive over-temperature alarm is to predict a high temperature condition (i.e. determine in advance a future condition) that would cause elevated temperature (or high heat) in the apparatus. In other words, the predictive alarm is configured to predict if the desired operation (e.g. therapy settings) of the apparatus will in future cause the device steady-state temperature to exceed a high-temperature threshold. A sustained high heat condition can cause sensors to error or malfunction, or may cause damage to the sensors. Additionally, or alternatively, high temperatures can cause the gases stream to increase in temperature or enthalpy, which could be unsafe for an end user. In some cases, if the temperature is too high, damage can be caused to components of the apparatus. For example, sustained high temperature can damage the blower or gases flow paths (i.e. pipes, connections, couplings, and/or seals) through the apparatus.

The predictive alarm method 200 compares the predicted steady-state temperature to a predetermined high-temperature-condition threshold. The high-temperature-condition threshold may be a stored data value or variable in memory associated with the controller. The high-temperature-condition threshold can be a configurable temperature parameter, such as a temperature value. The high-temperature-condition threshold can be a temperature parameter that is predetermined or selected based on the characteristics of the apparatus and/or the specific type of steady-state temperature being predicted for the alarm. For example, the high-temperature-condition threshold may be dependent on the particular temperature parameter that the predicted steady-state temperature represents. For example, the threshold for a predicted steady-state temperature representing the temperature of the gases flow might be different (higher or lower) compared to the threshold for a predicted steady-state temperature representing the temperature in a particular location with the housing of the apparatus.

By way of further example, in one configuration, the steady-state temperature represents a predicted temperature parameter relating to the temperature of flow of gases at a specified region or location within the apparatus. In this configuration, the high-temperature-condition threshold can be configured to represent a gases temperature safety threshold or value above which there is an increased risk of faulty operation, damage to the apparatus, and/or a safety risk to the user in relation to or caused by overheating of that gases flow. In another configuration, the steady-state temperature can be configured to represent a predicted temperature parameter relating to a specific location or region within a main housing of the apparatus, and the high-temperature-condition threshold can be configured to represent a housing temperature safety threshold or value above which there is an increased risk of faulty operation, damage to the apparatus, and/or a safety risk to the user in relation to or caused by overheating in that location or region of the main housing.

At step 206, the predictive alarm method 200 is configured to generate an alarm signal or predicted-high-temperature-condition signal if the predicted steady-state temperature exceeds the high-temperature-condition threshold. At step 208, the predictive alarm method 200 is configured to trigger or activate one or more alarms or alarm responses in response to the alarm signal or high-temperature-condition signal.

In one configuration, the alarm response may comprise generating an alarm notification. The alarm notification may be any one or more of the following types of alarms: audible, visual, tactile, and/or haptic. By way of example, the alarm notification may include the controller displaying an alarm message, notification, or indication on a display screen or a user interface of the apparatus.

In some configurations, the controller is configured to reject the set flow rate and disable the motor or reduce the motor to an idling speed if the alarm is generated.

In some configurations, the controller is configured to cap or reduce the motor speed to a maximum allowable motor speed and to generate an alarm message, notification or indication on a display screen or a user interface of the apparatus if an alarm is generated. In such a configuration, the maximum allowable motor speed can represent the highest motor speed, for the set flow rate, that the apparatus can sustain without the steady-state temperature exceeding the high-temperature-condition threshold.

In some configurations, the controller is configured to reduce or disable the power applied to or operation of one or more non-blower components of the apparatus to lower their heat output if the alarm is generated. In one example, the controller can be configured to reduce the power applied to and/or disable a heater plate of a humidifier and/or a heater wire of a patient interface conduit (e.g. breathing tube) if the alarm is generated. In another example, the controller is configured to reduce or disable the charging circuitry associated with a battery of the apparatus if the alarm is generated.

It will be appreciated that the controller may activate any one or more of the above example alarm responses in response to an alarm signal. As will be appreciated, the alarm response need not necessarily always trigger an alarm notification (such as a visual, tactile, and/or audible alarm). As described above, the alarm response executed by the controller may be to control the apparatus in a particular manner to address and/or avoid the predicted future over-temperate condition, such as varying or modifying operation settings or parameters, as discussed above. This may be done alone or in combination with generating an alarm notification.

The above generic description of the predictive over-temperature alarm 200 algorithm or process steps is generally applicable to the following first and second example embodiments, which provide example implementations and/or configurations of the overall alarm method 200. The first and second example embodiments demonstrate direct and indirect implementation methods of evaluating or predicting whether the steady-state temperature of the apparatus will exceed the predetermined high-temperature-condition threshold.

As will be appreciated, the predictive over-temperature alarm algorithms and/or processes are implemented by a controller or processor of the apparatus. The alarm algorithms and/or processes may be programmed as software or computer-readable instructions executable by the controller or processor of the apparatus. As will be appreciated, any required input signals and/or data (e.g. sensor signals and/or operational or therapy settings and/or user control inputs to the apparatus) used by the alarm algorithms can be retrieved or received by the controller. Likewise, any outputs or alarm responses generated by the alarm algorithms may be implemented by the controller via control signals or similar. It will also be appreciated that any relevant heat models utilised by the alarm algorithms may be stored and retrieved from electronic memory accessible by the controller and/or may be encoded in the software algorithms.

### 4. First example embodiment - predictive over-temperature alarm based on motor speed assessment

#### *Overview*

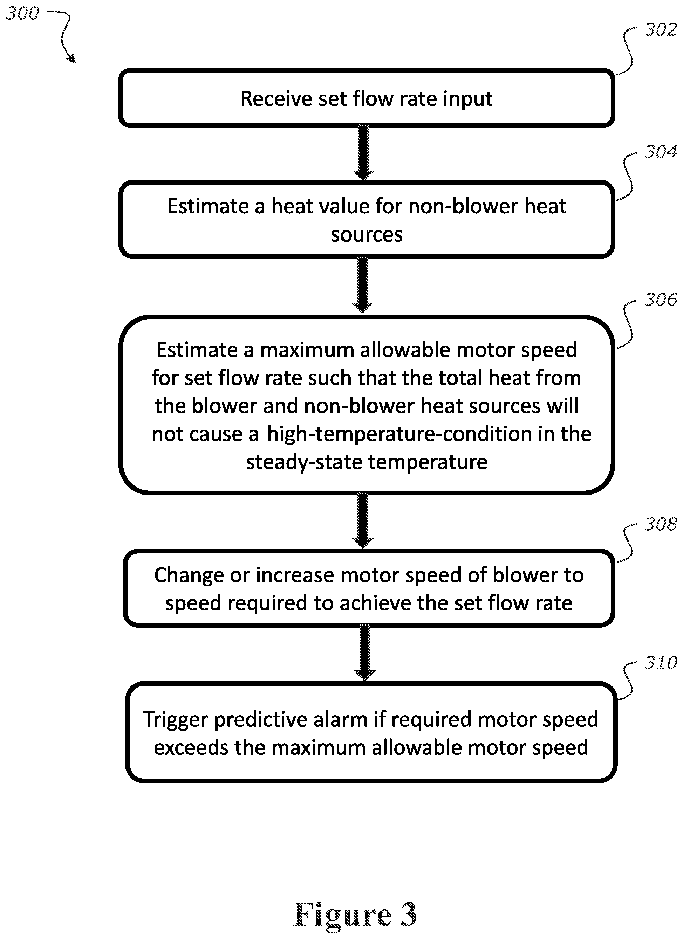

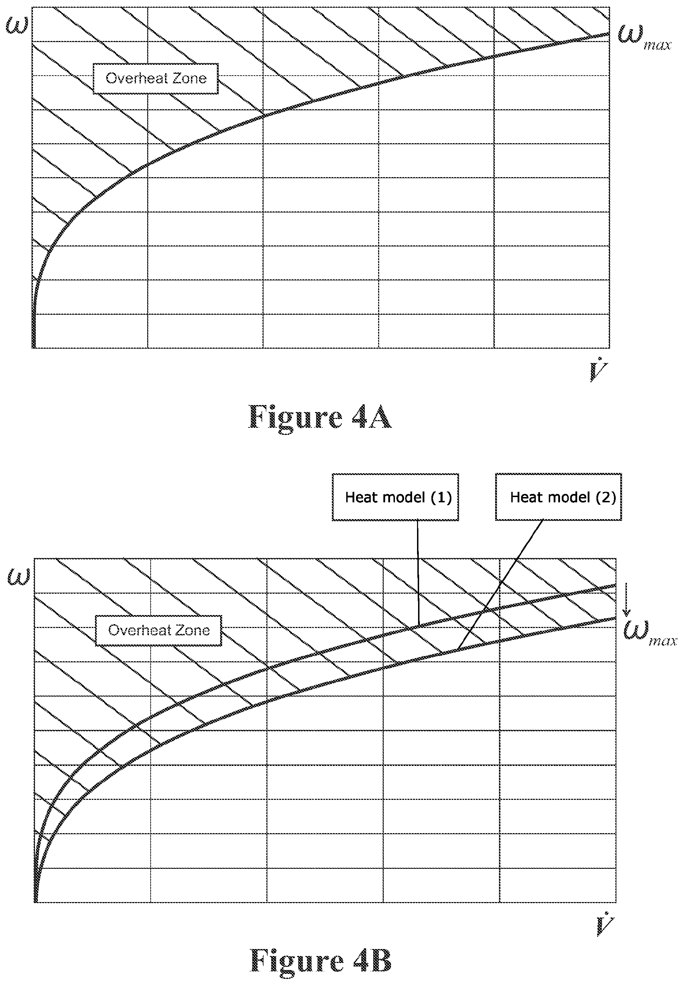

With reference to Figures 3-4C, a first example embodiment of the predictive over-temperature alarm 300 will be described in further detail. The predictive over-temperature alarm 300 in this first embodiment utilises an indirect alarm prediction based on comparing the required motor speed for a set flow rate input for a therapy session to an estimated maximum allowable motor speed that represents a speed at which there is a significant risk of the steady-state temperature of the apparatus exceeding a predetermined high-temperature condition. In brief, the controller or processor determines the maximum allowable motor speed value (for the current set flow rate) above which it predicts that there is a significant risk of the steady-state temperature of the apparatus exceeding a predetermined maximum.

In one configuration, the alarm algorithm determines or extracts the required motor speed for the set flow rate by allowing the apparatus controller to control motor speed of the blower to generate the set flow rate, using closed-loop feedback control as previously described. Once the motor speed required to generate the set flow rate is known, it is then compared to the predicted maximum allowable value. If the required motor speed exceeds this maximum allowable value (in order to achieve the current set flow), the processor will immediately activate an alarm response, without having to wait for the apparatus to actually heat up and exceed its predetermined maximum steady-state temperature. In one embodiment, the alarm response initiated is that the controller caps the motor speed back to the maximum allowable motor speed limit determined by the predictive over-temperature alarm algorithm. This will then limit the flow rate of the gases flow to below the target set flow rate, which may then trigger a 'flow below target' alarm. In other embodiments, the alarm algorithm may implement any one or more of the other alarm responses previously described.

In another configuration, the predictive over-temperature alarm is configured to determine or predict the maximum allowable motor speed value, for the set flow rate input of the therapy session, above which it predicts that there is a significant risk of the steady-state temperature of the apparatus exceeding a predetermined maximum or threshold. In this configuration, rather than then determining the required motor speed for the set flow rate and comparing that to the predicted limit, the predictive over-temperature alarm is causes the controller to cap or limit the motor speed of the blower to the predicted maximum allowable motor speed limit during the therapy session for that set flow rate input setting, such that the motor speed can never exceed the limit during the closed-loop feedback control of the blower. In this configuration, the predictive over-temperature alarm operates as a control algorithm or provides a limit input into the blower control such that it can't breach the maximum allowable motor speed.

In this embodiment, the controller is configured to predict if the steady-state temperature will exceed the high-temperature-condition threshold based on determining if the required motor speed for the set flow rate input exceeds a maximum allowable motor speed. The controller determines the maximum allowable motor speed and this represents the highest motor speed, for the set flow rate, that the apparatus can sustain without the steady-state temperature exceeding the high-temperature-condition threshold. In this embodiment, the maximum allowable motor speed is based on, or is a function of, a predetermined heat model. In effect, the maximum allowable motor speed, for the set flow rate, represents the high-temperature-condition threshold.

In this embodiment, the heat model is a function or threshold representing the maximum allowable motor speed for a range of set flow rates, and wherein the heat model is generated at least partly based on the high-temperature-condition threshold. As will be appreciated, the threshold may be defined by an equation or function that defines a limit or threshold as a function or based on motor speed and/or flow rate, and this threshold may be encoded in software code in the controller or memory accessible by the controller. The threshold may be represented graphically as a line (straight or curvilinear) on a plot or graph, such as that depicted and described later in Figures 4A and 4B.

In one configuration, the heat model represents the heat generated by the blower, which is a function of the flow rate and the motor speed. In another configuration, the heat model represents the heat generated by the blower, which is a function of the flow rate and the motor speed, and one or more non-blower heat sources. In one form, the heat model can be configured to represent the one or more non-blower heat sources with respective preset values representing the maximum heat that could realistically be generated by each of the one or more non-blower heat sources during operation. In another form, the heat model can be configured to represent the one or more non-blower heat sources with one or more respective predetermined or configurable safety values that represent the maximum heat predicted to be generated by the respective heat sources in the desired therapy session.

In one configuration, the heat model is configured to model heat (energy) and/or temperature rise caused by one or more heat sources within the apparatus. In particular, the heat model is configured to model the heat produced by one or more heat sources in the apparatus, and then models the temperature rise that will or could occur in the apparatus as a result of this heat in and/or flowing through the apparatus. The determination of the maximum-allowable motor speed for a set flow rate input from the heat model may be considered a re-arrangement of the heat model. In one configuration, the heat model involves or comprises calculating temperature rise based on modelling the heat energy of one or more heat sources within the apparatus.

In one configuration of this embodiment, the maximum allowable motor speed for a range of flow rates is represented or defined by a predetermined threshold line plotted on a motor speed versus flow rate graph. The area above the threshold line representing a high-temperature-condition zone. In this configuration, the controller is configured to predict if the steady-state temperature will exceed the high-temperature-condition threshold by determining if the required motor speed and set flow rate combination is in the high-temperature-condition zone.

## *Example implementation process*

A more detailed example of an implementation of the first embodiment predictive over-temperature alarm 300 will now be described.

Referring to Figure 3, the predictive alarm process implemented by the controller of the apparatus commences by receiving or retrieving the set flow rate input for a new or desired therapy session, or receives an updated or changed set flow rate input or setting for a current therapy session, as shown at 302. As previously described, in this embodiment, the predictive alarm algorithm is implemented as software code, such as computer-readable instructions executable by the controller or a processor within the apparatus.

The controller then estimates a first heat value representing the heat contributing to the steady-state temperature by one or more non-blower heat sources as shown at 304. By way of example, the controller estimates the heat that would be added to the apparatus if the temperatures of one or more predetermined or selected non-blower components (e.g. a backup battery, a heater plate of the humidifier) within the apparatus increased to their realistic maximums, and/or the heat from the environmental sources (e.g. the ambient air) increased to its realistic maximum. In one form, the alarm algorithm stores fixed individual maximum heat or temperature rise values associated with the one or more non-blower components for use in the heat model. In some configurations, the predictive alarm 300 can be configured to take into account all or at least the majority of non-blower heat sources in and/or associated with the apparatus. It will be appreciated that the predictive alarm 300 need not necessarily consider the heat energy from every non-blower component and/or environmental variable, but may consider at least one or more of the significant non-blower heat sources that will contribute the majority of the heat to the apparatus during operation.

In alternative embodiments, the algorithm may use real-time determined temperature and/or operation values for some non-blower heat sources for those that are considered stable across a therapy session, instead of using fixed predetermined realistic maximum values. For example, any one or more of the following real-time values could be used to represent the non-blower heat sources in the heat model: real-time temperature sensor values in the apparatus, humidifier heater plate power or current, heated breathing tube power or current, and/or battery power or current, depending on which non-blower components are used in the heat model. By way of further explanation, the apparatus may have one or more temperature sensors, such as an ambient temperature sensor and some of the non-blower components (e.g. humidifier heater plate, heated breathing tube, battery) may have a temperature sensor associated with them to sense their temperatures. In some configurations, these sensed temperatures may be fed into the predictive alarm algorithm in real-time as an alternative to the heat model using predetermined maximum temperature values for one or more non-blower components in the heat model.

Next, the controller estimates a maximum allowable motor speed of the blower corresponding to a second heat value representing the heat contributing to the steady-state temperature by the blower such that the aggregate of the first heat value and second heat value do not cause the steady-state temperature to exceed the high-temperature-condition threshold as shown at 306. By way of example, in one configuration, the relationship between the motor speed-flow combination and the heat that is likely to be generated by the blower is known for the apparatus and stored in memory of or accessible by the controller. Generally, the higher the motor speed required to achieve a given set flow, the more heat generated by the blower. Using this known and stored relationship, the controller estimates the maximum motor speed that can be sustained (for the current set flow) without the sum of the heat generated by the blower (second heat value) and the heat generated by the non-blower components and environmental variables (first heat value) causing the steady-state device temperature to exceed its maximum (predetermined high-temperature-condition threshold).

In another configuration, steps 304 and 306 may be combined. In particular, the alarm algorithm may use a stored heat model (e.g. equation or function) that represents a merged or combined model of the heat energy and/or temperature rise caused by the one or more non-blower components and the blower, for the set flow rate input. As above, a maximum heat value or temperature rise may be assumed in the model for the non-blower components, or they may be represented as individual or combined variables which vary in heat generation or temperature rise based on the set flow rate input.

Once the maximum motor speed has be determined, the controller is configured to increase or change the motor speed of the blower to the motor speed required for the blower to generate or achieve the set flow rate as shown at 308. In particular, the controller uses closed-loop control to vary the motor speed to achieve the set flow rate, using a flow rate sensor and/or motor speed sensor. This enables the controller to dynamically determine the required motor speed for the set flow rate input. As will be appreciated, depending on the apparatus configuration and/or components being used (e.g. type of patient interface), the specific motor speed required to generate the set flow rate may vary from apparatus to apparatus or from user to user, so is more accurately determined dynamically in real-time. In an alternative configuration, it will be appreciated that a model or estimate of the relationship between motor speed and flow rate can be stored in the controller, and this model can be used to extract the required motor speed for the set flow rate input, rather than determining the required motor speed dynamically, but the model output will be an estimate.

Once the required motor speed is determined (whether dynamically or estimated), the controller then predicts if the steady-state temperature of the apparatus will exceed the high-temperature-condition threshold based on the determined maximum allowable motor speed and the determined required motor speed as shown at 308. In particular, if the required motor speed exceeds the maximum allowable motor speed, then the prediction is made that the steady-state temperature of the apparatus will at some stage exceed the predetermined high-temperature-condition threshold, and the alarm is triggered at 310. As explained previously, the controller may initiate one or more alarm responses in response to the alarm being triggered to combat or avoid the predicted over-temperature condition.

### *Heat model*

Referring to Figures 4A-4C, the heat model configuration utilised in the first embodiment predictive over-temperature alarm 300 will now be explained in further detail, by way of example.

As described, at the beginning of a new therapy session, or during a therapy session if the set flow rate is changed, or if the apparatus is stopped during a therapy session and then restarted, the controller implements the predictive over-temperature algorithm to predict whether the new or updated set flow rate can be achieved, and maintained, without the apparatus steady-state temperature exceeding a predetermined maximum (high-temperature-condition threshold).

The predictive alarm 300 algorithm enables the controller to estimate a motor speed value $\omega_{max}$ for any given flow rate ($\dot{V}$), above which the blower will generate enough heat to cause the steady-state apparatus temperature to exceed its maximum value ($T_{max}$), which represents the configurable high-temperature-condition threshold. Generally, the higher the motor speed required to achieve a given flow, the more heat is generated by the blower. In this embodiment, one example form of equation or heat model for determining $\omega_{max}$ is:

$$\omega_{max} = f\left(k\sqrt[3]{\dot{V}}\right) \qquad (1)$$

where $k$ is a constant related to the flow path geometry of the apparatus. When visualized on a flow-motor speed graph or plot, $\omega_{max}$ may appear as shown in Figure 4A. As shown in Figure 4A, the value of $\omega_{max}$ changes as the set flow rate changes, and the area above the curve representing $\omega_{max}$ over the flow rate ($\dot{V}$) range is the overheat or over-temperature zone. In one configuration of the heat model, if the required motor speed for a set flow rate (i.e. the motor speed and flow rate combination) is in the overheat zone, then the predictive alarm will trigger. In this configuration of the heat model (1), the blower is considered as the only heat source. However, as explained in the following, in other configurations, a more complete heat model is used which also takes into account at least some or all of the other non-blower heat sources, such as other apparatus components (e.g. a battery, a heater plate of a humidifier, etc) and/or environmental (e.g. ambient temperature) conditions.

As discussed above, it is not just heat from the blower that can contribute to the steady-state temperature of the apparatus exceeding $T_{max}$. For example, heat from a battery power source and the ambient environment can also contribute, as well as any other heat sources considered or selected to be significant and included in the heat model. As the temperatures of the battery, ambient environment, and other selected heat sources, increase, less heat can be generated by the blower before $T_{max}$ is exceeded. Therefore, an example of a more complete form of equation or heat model for determining $\omega_{max}$ is:

$$\omega_{max} = f\left(\sqrt[3]{\dot{V}, T_{ambient}, T_{battery}, ... T_{other\ heat\ sources}}\right) \qquad (2)$$

where $T_{ambient}$ is the temperature of the ambient environment, $T_{battery}$ is the temperature of the battery within the apparatus (if one is present), and $T_{other\ heat\ sources}$ represents the temperature contributed by one or more other selected heat sources within the apparatus that are significant contributors to the steady-state temperature of interest. In one configuration of this heat model (2), the values for one or more of the non-blower heat source variables may be preset or predetermined. For example, the values for $T_{ambient}$, $T_{battery}$, ... $T_{other\ heat\ sources}$ may be preset to their maximum realistic values, in order to obtain a conservative, worst-case scenario value for $\omega_{max}$. Referring to Figure 4B, when visualized on a flow-motor speed graph or plot, the $\omega_{max}$ curve, calculated with the more complete heat model (2) is lower than the $\omega_{max}$ curve calculated with the less-complete blower-only heat model (1).

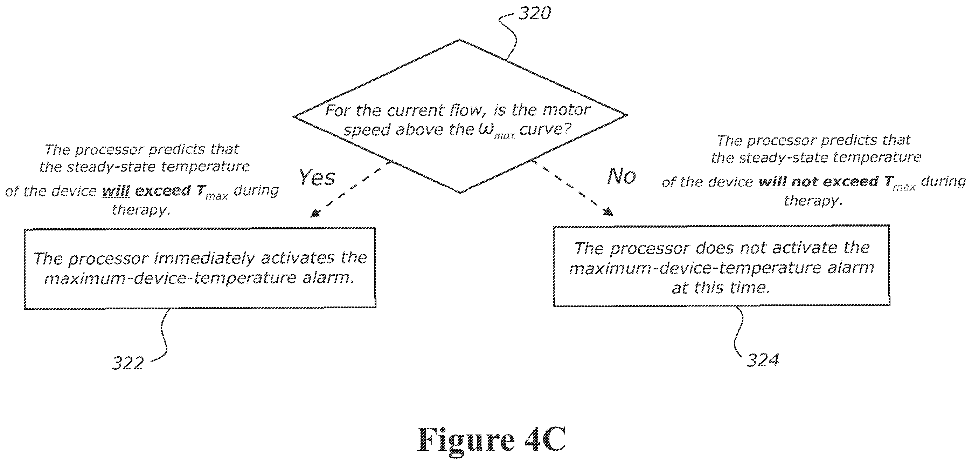

Referring to Figure 4C, the predictive alarm algorithm implemented by the controller determines the $\omega_{max}$ for the set flow rate input based on, or as a function of, the heat model. The algorithm then determines whether the required or current motor speed for that set flow rate is above the $\omega_{max}$ curve of the heat model as shown at 320. If the required motor speed is above the $\omega_{max}$ curve, then the controller predicts that the steady-state temperature of the device will exceed the $T_{max}$ of the apparatus during the therapy session, and triggers the alarm as shown at 322. If the required motor speed is at or below $\omega_{max}$ curve, the controller predicts that the steady-state temperature of the apparatus will not exceed the $T_{max}$ of the apparatus during the therapy session, and does not trigger the alarm as shown at 324.

It will be appreciated that the values for the non-blower heat sources included in the more complete heat model (2) may be fixed predetermined values as described above, or alternatively in other configurations may be dynamic variables that are determined in real-time to update the heat model during operation. For example, in some configurations, some components and/or variables may be determined in real-time, particularly those that may be considered as being more likely to be stable throughout a therapy session. By way of example, the value for $T_{ambient}$ might be determined by an ambient temperature sensor of or operatively connected to the apparatus, and the sensed ambient temperature may be used in the heat model if it is likely that this ambient temperature will be relatively stable throughout the therapy session, which may be the case in use-scenarios in which the apparatus is being used in a climate-controlled room or environment. Likewise, it is possible that other non-blower heat source components of the apparatus in the heat model could be determined in real-time by temperature sensors associated with the components of the apparatus to update the heat model during operation of the apparatus..

The more complete heat model (2) described above, which takes into account the blower heat source as well as one or more other non-blower heat sources of or associated with the apparatus, is provided by way of example only. It will be appreciated that the heat model can be configured to take into account any desired one or more of the non-blower heat sources or components of or associated with the apparatus. In one configuration, the heat model can be configured to model all or at least the majority of the significant heat sources relevant to the steady-state temperature of interest to the predictive alarm, and in other configurations the heat model may be configured to take into account one or more selected heat sources. The heat model can be customised and/or configured based on various factors including, but not limited to, one or more of the following: the specific configuration and characteristics of the apparatus, apparatus modes of operation, peripheral components connected to the apparatus such as patient interface conduits and/or interfaces (cannula, masks etc), and/or the desired sensitivity and/or safety factor desired for the predictive alarm.

## 5. Second example embodiment - determining if predicted steady-state temperature of apparatus exceeds high-temperature-condition threshold

### *Overview*

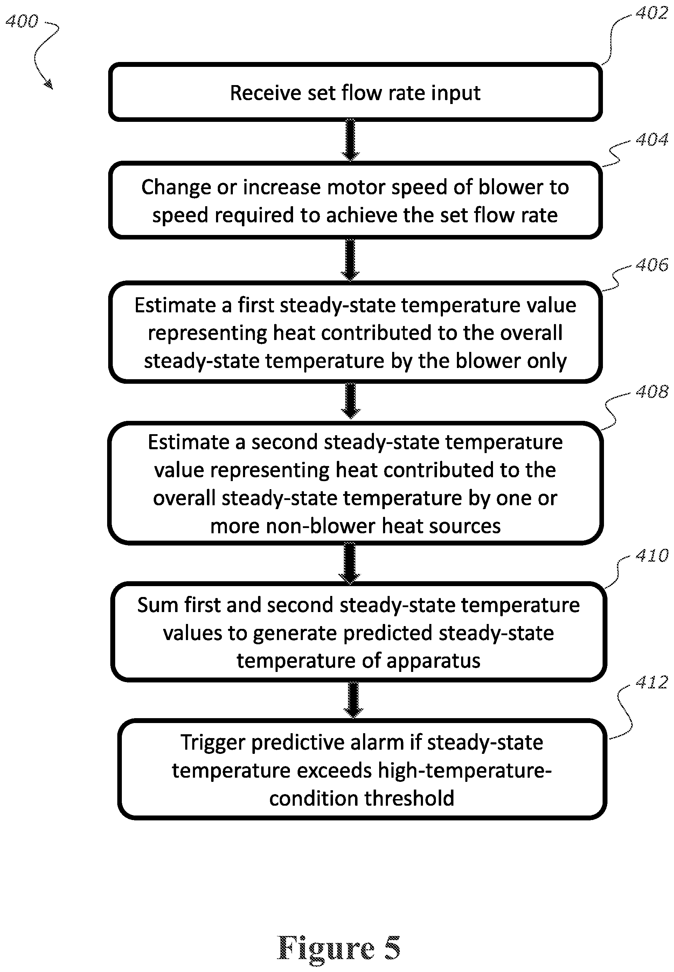

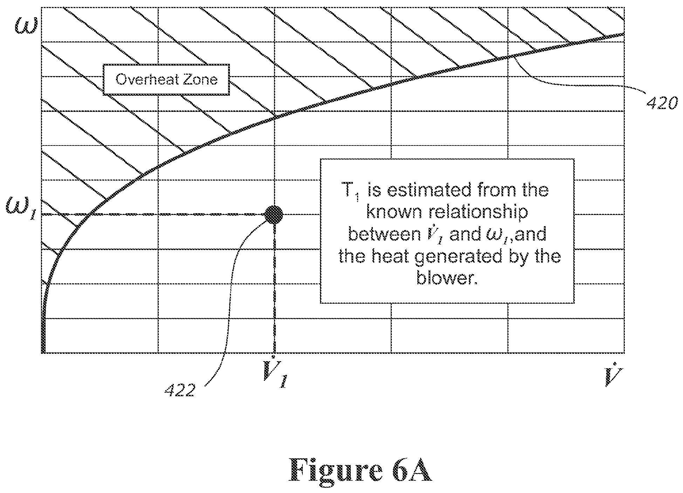

With reference to Figures 5-6B, a second example embodiment of the predictive over-temperature alarm 400 will be described in further detail. The predictive over-temperature alarm 400 in this second embodiment utilises a direct alarm prediction in which the steady-state temperature of the apparatus is predicted and compared to a maximum device temperature (high-temperature-condition threshold). In this second embodiment, the controller or processor predicts the highest steady-state temperature that the apparatus could reach given the current set flow and the motor speed required to achieve it. If this predicted steady-state temperature is above a predetermined maximum temperature (high-temperature-condition threshold), the controller will immediately activate an alarm, without having to wait for the apparatus to actually heat up and exceed its maximum steady-state temperature.

In this embodiment, the controller is configured to predict if the steady-state temperature will exceed the high-temperature-condition threshold is based on predicting the steady-state temperature as a function of the required motor speed and set flow rate, and determining if the predicted steady-state temperature exceeds the high-temperature-condition threshold.

In this embodiment, predicting the steady-state temperature comprises extracting an estimate from a predetermined heat model. In one configuration, the heat model represents the heat generated by the blower, which is a function of the flow rate and the motor speed. In another configuration, the heat model represents the heat generated by the blower, which is a function of the flow rate and the motor speed, and one or more non-blower heat sources. In one form, the heat model can be configured to represent the one or more non-blower heat sources with respective preset values representing the maximum heat that could realistically be generated by each of the one or more non-blower heat sources during operation. In another form, the heat model can be configured to represent the one or more non-blower heat sources with one or more respective predetermined or configurable safety values that represent the maximum heat predicted to be generated by the respective heat sources in the desired therapy session.

In this embodiment, the controller is configured to predict if the steady-state temperature of the apparatus will exceed the high-temperature-condition threshold by predicting the steady-state temperature based on, or as a function of, the predetermined heat model, the set flow rate, and the required motor speed. Following this, the controller determines whether the predicted steady-state temperature exceeds the high-temperature-condition threshold. In one configuration, the heat model can generate a temperature variable representing the predicted steady-state temperature based at least on the set flow rate and the required motor speed such that the heat model is at least based on the heat generated by the blower. In another configuration, the heat model generates a temperature variable representing the predicted steady-state temperature based at least on the set flow rate, the required motor speed, and one or more other parameters representing non-blower heat-sources of or associated with the apparatus that will contribute to the steady-state temperature of the apparatus. In either configuration, the controller is configured to determine if the temperature variable representing the predicted steady-state temperature exceeds the high-temperature-condition threshold, and generates an alarm if the threshold is exceeded.

### *Example implementation process*

A more detailed example of an implementation of the second embodiment predictive over-temperature alarm 400 will now be described.

Referring to Figure 5, the predictive alarm process implemented by the controller of the apparatus commences by receiving or retrieving the set flow rate input for a new or desired therapy session as shown at 402.

Next, the controller then increases or changes the motor speed of the blower to the motor speed required for the blower to generate or achieve the set flow rate as shown at 404. This step is similar to that described at 308 with regard to the first embodiment, and the same options and configurations are applicable in this embodiment.

Once the required motor speed is determined for the set flow rate, the controller is then configured to estimate a first steady-state temperature value or component representing the heat contributed to the steady-state temperate by the blower only as shown at 406. By way of example, the relationship between the motor speed-flow combination and the heat that is likely to be generated by the blower is known for the apparatus and stored in memory or accessible by the controller. Generally, the higher the motor speed required to achieve a given set flow, the more heat generated by the blower. Using this known and stored relationship, and the current motor-speed flow combination, the controller estimates the steady-state temperature that the apparatus would reach if the only source of heat was the blower.

Next, the controller is configured to estimate a second steady-state temperature value or component representing the heat contributed to the steady-state temperature by one or more non-blower heat sources as shown at 408. By way of example, the controller estimates how far the steady-state temperature of the apparatus could increase (above the temperature estimated from the first steady-state temperature value at 406) if one or more non-blower heat sources (e.g. a heater plate of the humidifier, a backup battery, ambient air) increased to their realistic maximums. It will be appreciated that the predictive alarm 400 need not necessarily consider the heat energy from every non-blower component and/or environmental variable, but may consider at least one or more of the significant non-blower heat sources that will contribute the majority of the heat to the apparatus during operation. In alternative embodiments, the algorithm may use real-time determined temperature values for some non-blower heat sources for those that are considered stable across a therapy session, instead of using fixed predetermined realistic maximum values.

The controller predicts the steady-state temperature of the apparatus by aggregating or summing the first steady-state temperature value and second steady-state temperature value as shown at 410. Following this, the controller then triggers the predictive alarm if the predicted steady-state temperature exceeds the high-temperature-condition threshold, as shown at 412. As explained previously, the controller may initiate one or more alarm responses in response to the alarm being triggered to combat or avoid the predicted over-temperature condition.

### *Heat model*

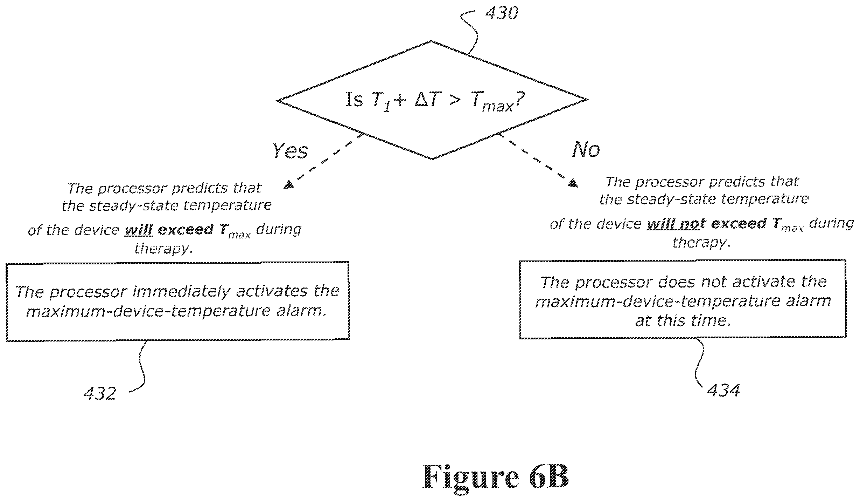

Referring to Figures 6A and 6B, the heat model configuration utilised in the second embodiment predictive over-temperature alarm 400 will now be described in further detail, by way of example.

Given the known relationship between the flow ($\dot{V}_1$) and motor speed ($\omega_1$), and the heat generated by the blower, the predictive alarm 400 enables the controller to estimate the steady-state temperature ($T_1$) that the apparatus would reach during a therapy session if the only heat source was the blower, and this is represented by the following:

$$T_1 = f\left(\dot{V}_1, \omega_1\right) \qquad (3)$$

Referring to Figure 6A, the combination of the set flow rate ($\dot{V}_1$) and motor speed ($\omega_1$) required to achieve the flow rate is represented as the first steady-state temperature value $T_1$ shown at 422, and represents the heat generated by the blower only. If this $T_1$ 422, as represented by the flow-motor speed combination ($\dot{V}_1, \omega_1$), is in the overheat zone above the high-temperature-condition threshold line 422, the predictive alarm may immediately trigger the alarm. Alternatively, if the initial steady-state temperature value from the blower only, $T_1$, is below the high-temperature-condition threshold line 422 (i.e. out of the overheat zone), the predictive alarm 400 proceeds to the next step of assessing heat contributed by non-blower heat sources, as described further below.

The predictive alarm 400 enables the controller to predict how far the steady-state temperature of the apparatus could increase above the first steady-state temperature $T_1$ during the therapy session (without any changes to the flow rate or motor speed). This temperature increase ($\Delta T$) could be caused by other, non-blower heat sources such as, but not limited to, a battery and the ambient air, or any of the other non-blower heat sources discussed previously. As such the heat model may represent this second steady-state temperature value associated with the non-blower heat sources as:

$$\Delta T = f\left(T_{ambient}, T_{battery}, \dots T_{other\ heat\ sources}\right) \qquad (4)$$

In one configuration, as described previously, the predictive alarm algorithm 400 presets the values of $T_{ambient}$, $T_{battery}$, ... $T_{other}$ *heat sources* to their maximum realistic values, in order to obtain a conservative, worst-case scenario value for $\Delta T$.

Once $T_1$ and $\Delta T$ have been determined, the algorithm enables the controller to compare their sum, which represents the predicted highest steady-state temperature the apparatus could reach during the therapy session (given all of the likely heat sources accounted for in the heat model), to the maximum allowable apparatus temperature $T_{max}$ (high-temperature-condition threshold). With reference to Figure 6B, the predictive alarm algorithm 400 compares whether the steady-state temperature prediction represented by the sum of $T_1$ and $\Delta T$ is greater than the high-temperature-condition threshold $T_{max}$ as shown at 430. If the controller determines that the predicted steady-state temperature is greater than the threshold, then the controller triggers the alarm as shown at 432. If the controller determines that the predicted steady-state temperature will not exceed the threshold, then the alarm is not triggered as shown at 434.

### 6. *Third example embodiment* - *Pressure therapy apparatus with predictive over-temperature alarm*

The embodiments above describe implementation of the predictive over-temperature alarm in the context of a respiratory apparatus that is a flow therapy apparatus or is operable in a flow therapy mode. As mentioned, the principles of the predictive over-temperature alarm may also be implemented in other types of respiratory apparatus or different therapy modes.

By way of example, an implementation of the predictive over-temperature alarm in the context of a respiratory apparatus in the form of a pressure therapy apparatus or a respiratory apparatus that is operating in a pressure therapy mode, will now be described. By way of example, such pressure therapy modes may include, but are not limited to, continuous positive airway pressure (CPAP), Bi-level positive airway pressure (BiPAP), Bubble CPAP (BCPAP), Non-invasive ventilation (NIV). It will be appreciated that the theory and principles of operation described from the earlier embodiments may be applied and adapted into a pressure therapy apparatus, as explained further below.

In the pressure therapy apparatus (or when a respiratory apparatus is operating in a pressure therapy mode), the user selects or inputs a target pressure setting or settings relating to the pressure of the gases stream to be delivered to the user in the therapy session via a sealed patient interface, such as a full-face mask. The pressure setting or settings might be a single pressure setting in the context of CPAP therapy, or an inspiratory pressure setting and expiratory pressure setting in the context of BiPAP therapy for example. The controller implemented the predictive over-temperature alarm then determines a motor speed or motor speed range of the blower required to achieve the set pressure level or levels. Using any of the heat models previously described in the former embodiments, the predictive alarm algorithm then predicts if an over-temperature condition will arise in which the steady-state temperature of the apparatus will exceed a high-temperature threshold condition and initiates one or more alarm responses if it is predicted that the current pressure or operating settings will cause the threshold to be exceeded at some point in the future.

In one configuration, the predictive over-temperature alarm calculates a maximum allowable motor speed or motor speed range based on the heat model and pressure settings. Above this allowable motor speed or motor speed range, the steady-state temperature of the apparatus is predicted as exceeding a high-temperature threshold condition. The controller then treats the determined allowable motor speed or motor speed range as a threshold or limits, and restricts the motor speed of the blower from exceeding the allowable motor speed threshold or limits during the pressure therapy session, to avoid an overheating condition in the apparatus.

In another configuration, the predictive over-temperature alarm may calculate a maximum allowable motor speed or motor speed range based on the heat model and pressure settings as above, and then alarm or initiate an alarm response if the measured or sensed motor speed of the blower exceeds the maximum allowable motor speed limit for a predetermined time period. The predetermined time period may be selected or determined to correspond to when the steady-state temperature is likely to exceed the high-temperature threshold condition.

It will be appreciated that a respiratory apparatus can be configured and operable in one or more therapy modes. For example, a respiratory apparatus may be selectively operable to provide flow therapy, such as nasal high flow therapy, and/or alternatively pressure therapy such as CPAP, BiPAP, or BPAP for example. In such apparatus, the predictive over-temperature alarm may be implemented in any one or more or all of these therapy modes.

### 7. *Over-temperature detection system in the respiratory apparatus*

In any of the above example embodiments of the respiratory apparatus, whether flow therapy, pressure therapy, or both, the apparatus may also be configured with a system for detection of actual over-temperature conditions during operation. This actual over-temperature detection system is in addition and independent to the predictive over-temperature alarm algorithm.

By way of example, the over-temperature detection system of the respiratory apparatus is configured to monitor the internal apparatus temperature via one or more temperature sensors in the apparatus. If any one or more of the temperature sensors exceeds a relevant over-temperature condition threshold, then an alarm will trigger or activate. In one configuration, the alarm will cause a notification or message to generate and present on the display screen of the apparatus. In the context of a flow therapy session, the alarm notification may indicate that the target flow rate is too high, and this indicates that the operation of the apparatus at the current flow rate settings has caused the internal apparatus temperature to exceed a safety threshold or high-temperature condition. By way of example only, the alarm may trigger if the temperature is sensed as being at or over a first temperature threshold for a first time period, or at or over a second temperature threshold for a second time period, where the first temperature threshold is lower than the second temperature threshold and the first time period is longer than the second time period. For example, the alarm may be configured to trigger if the sensed temperature is 70 degrees Celsius for 60 seconds, or over 85 degrees Celsius for 10 seconds. It will be appreciated that there may be multiple cascading temperature thresholds and corresponding time periods, with higher thresholds having shorter time periods.

In one configuration, if the alarm is triggered, an alarm message may be presented to the user indicating that they should reduce the flow rate setting, and/or reduce the humidifier heater plate power, and/or reduce the heated breathing tube power. Alternatively, the controller may automatically reduce or control the flow rate and/or power of the humidifier heater plate and/or heated breathing tube to cool the apparatus. The over-temperature alarm may clear or reset once the device has cooled sufficiently as sensed or measured by the one or more temperature sensors.

In some configurations, the respiratory apparatus may comprise one or more temperature sensors, such as thermistors, in the blower module. The thermistors in the blower module monitor the temperature of the blower. In such configurations, if the sensed temperature of the blower exceeds a temperature threshold, then the motor speed of the blower is reduced to a lower speed or the motor of the blower is stopped for a period of time until the blower temperature has sufficiently reduced to an acceptable level based on a threshold. In this configuration, the blower thermistors are used as a safety feature to ensure that if the actual temperature in the blower exceeds a threshold, the blower motor speed is reduced or the motor halted or switched off to prevent damage to the blower.

## 8. _General_

Furthermore, embodiments may be implemented by hardware, software, firmware, middleware, microcode, or any combination thereof. When implemented in software, firmware, middleware or microcode, the program code or code segments to perform the necessary tasks may be stored in a machine-readable medium such as a storage medium or other storage(s). A processor may perform the necessary tasks. A code segment may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

In the foregoing, a storage medium may represent one or more devices for storing data, including read-only memory (ROM), random access memory (RAM), magnetic disk storage mediums, optical storage mediums, flash memory devices and/or other machine readable mediums for storing information. The terms "machine readable medium" and "computer readable medium" include, but are not limited to portable or fixed storage devices, optical storage devices, and/or various other mediums capable of storing, containing or carrying instruction(s) and/or data, including non-transitory mediums.

The various illustrative logical blocks, modules, circuits, elements, and/or components described in connection with the examples disclosed herein may be implemented or performed with a general purpose processor, a digital signal processor (DSP), an application specific integrated circuit (ASIC), a field programmable gate array (FPGA) or other programmable logic component, discrete gate or transistor logic, discrete hardware components, or any combination thereof designed to perform the functions described herein. A general purpose processor may be a microprocessor, but in the alternative, the processor may be any conventional processor, controller, microcontroller, circuit, and/or state machine. A processor may also be implemented as a combination of computing components, e.g., a combination of a DSP and a microprocessor, a number of microprocessors, one or more microprocessors in conjunction with a DSP core, or any other such configuration.

The methods or algorithms described in connection with the examples disclosed herein may be embodied directly in hardware, in a software module executable by a processor, or in a combination of both, in the form of processing unit, programming instructions, or other directions, and may be contained in a single device or distributed across multiple devices. A software module may reside in RAM memory, flash memory, ROM memory, EPROM memory, EEPROM memory, registers, hard disk, a removable disk, a CD-ROM, or any other form of storage medium known in the art. A storage medium may be coupled to the processor such that the processor can read information from, and write information to, the storage medium. In the alternative, the storage medium may be integral to the processor. Additionally, the features described herein may be implemented in software, firmware, hardware, and/or any combination thereof.

**EP 4 304 692 B1**

In its various aspects, the invention can be embodied in a computer-implemented process, a machine (such as an electronic device, or a general purpose computer or other device that provides a platform on which computer programs can be executed), processes performed by these machines, or an article of manufacture. Such articles can include a computer program product or digital information product in which a computer readable storage medium containing computer program instructions or computer readable data stored thereon, and processes and machines that create and use these articles of manufacture.

The foregoing description of example embodiments includes example forms and configurations. Modifications may be made without departing from the scope of the disclosure and/or accompanying claims.

**Claims**

1. A respiratory support apparatus (10) that is configured to provide a flow of gases to a user for respiratory flow therapy comprising:

   a flow generator (11) that is operable to generate a flow of gases;
   a controller (13) that is operatively connected to the flow generator (11) and operable to control a flow rate of the flow of gases by controlling a motor speed of the flow generator (11), wherein the controller (13) is configured to:

      receive (202) an input that represents a set flow rate at which the apparatus is to generate the flow of gases for a desired therapy session comprising respiratory flow therapy;
      predict (204) if a steady-state temperature of the apparatus will exceed a high-temperature-condition threshold, based at least partly on a motor speed required to generate the set flow rate; and
      generate (206) a predicted-high-temperature-condition signal if the high-temperature-condition threshold is exceeded based on the prediction.

2. A respiratory support apparatus according to claim 1 wherein predicting (204) if the steady-state temperature will exceed the high-temperature-condition threshold is based on determining if the required motor speed exceeds a maximum allowable motor speed, wherein the maximum allowable motor speed represents the highest motor speed, for the set flow rate, that the apparatus can sustain without the steady-state temperature exceeding the high-temperature-condition threshold.

3. A respiratory support apparatus according to claim 2 wherein the maximum allowable motor speed is based on, or is a function of, a predetermined heat model, and optionally wherein:

   the heat model represents the heat generated by the flow generator (11), which is a function of the flow rate and the motor speed; or
   the heat model represents the heat generated by the flow generator (11), which is a function of the flow rate and the motor speed, and one or more non-flow generator heat sources.

4. A respiratory support apparatus according to claim 3 wherein the heat model is configured to represent the one or more non-flow generator heat sources with respective preset values representing an estimate of the maximum heat that could be generated by each of the one or more non-flow generator heat sources during operation.

5. A respiratory support apparatus according to claim 1 wherein predicting (204) if the steady-state temperature will exceed the high-temperature-condition threshold is based on:

   predicting the steady-state temperature as a function of the required motor speed and set flow rate; and
   determining if the predicted steady-state temperature exceeds the high-temperature-condition threshold, and optionally wherein predicting the steady-state temperature comprises determining an estimate from a predetermined heat model.

6. A respiratory support apparatus according to claim 1 wherein the controller (13) is configured to predict (204) if the steady-state temperature of the apparatus will exceed the high-temperature-condition threshold by:
   determining if the required motor speed exceeds a maximum allowable motor speed for the set flow rate, the maximum allowable motor speed representing the high-temperature-condition threshold, and optionally wherein the maximum allowable motor speed for the set flow rate is determined based on, or is a function of, a predetermined heat model.

7. A respiratory support apparatus according to claim 1 wherein the controller (13) is configured to predict (204) if the steady-state temperature of the apparatus will exceed the high-temperature-condition threshold by:

predicting the steady-state temperature based on, or as a function of, a predetermined heat model, the set flow rate, and the required motor speed; and
determining if the predicted steady-state temperature exceeds the high-temperature-condition threshold.

8. A respiratory support apparatus according to claim 7 wherein the heat model generates a temperature variable representing the predicted steady-state temperature based at least on the set flow rate, the required motor speed, and one or more other parameters representing non-flow generator heat-sources of or associated with the apparatus (10) that will contribute to the steady-state temperature of the apparatus (10).

9. A respiratory support apparatus according to any one of claims 3-8 wherein the heat model represents one or more heat sources of or associated with the apparatus, including at least representing the heat generated by the flow generator (11) as a function of motor speed and flow rate, and optionally wherein the heat model further represents the heat generated by one or more non-flow generator heat sources of or associated with the apparatus that will contribute to the steady-state temperature of the apparatus (10).

10. A respiratory support apparatus according to any one of claims 1-9 wherein the controller (13) is configured to receive the set flow rate input for the desired therapy session when the apparatus (10) is at start-up or standby with the flow generator stopped or idling at a non-therapy speed prior to the desired therapy session beginning; and/or wherein the controller (13) is configured to receive the set flow rate input while the apparatus (10) is already operating in a current therapy session at a first flow rate, and the set flow rate input is a different second flow rate relating to the new desired therapy session.

11. A respiratory support apparatus according to claim 1 wherein the controller (13) is configured to predict if the steady-state temperature of the apparatus (10) will exceed the high-temperature-condition threshold based at least partly on the required motor speed by:

estimating a first heat value representing the heat contributing to the steady-state temperature by one or more non-flow generator heat sources;
estimating a maximum allowable motor speed of the flow generator (11) corresponding to a second heat value representing the heat contributing to the steady-state temperature by the flow generator (11) such that the aggregate of the first heat value and second heat value do not cause the steady-state temperature to exceed the high-temperature-condition threshold;
increasing or changing the motor speed of the flow generator (11) to the motor speed required for the flow generator to generate the set flow rate so as to thereby determine the required motor speed for that set flow rate; and
predicting that the steady-state temperature of the apparatus (10) will exceed the high-temperature-condition threshold if the determined required motor speed is above the estimated maximum allowable motor speed.

12. A respiratory support apparatus according to claim 1 wherein the controller (13) is configured to predict if the steady-state temperature of the apparatus will exceed a high-temperature-condition threshold based at least partly on the required motor speed by:

increasing or changing (404) the motor speed of the flow generator to the motor speed required for the flow generator to generate the set flow rate so as to thereby determine the required motor speed for that set flow rate;
estimating (406) a first steady-state temperature value representing the heat contributed to the steady-state temperate by the flow generator;
estimating (408) a second steady-state temperature value representing the heat contributed to the steady-state temperature by one or more non-flow generator heat sources;
predicting (410) the steady-state temperature of the apparatus by aggregating the first steady-state temperature value and second steady-state temperature value; and
evaluating (412) if the predicted steady-state temperature exceeds the high-temperature-condition threshold.

13. A respiratory support apparatus according to any one of claims 3, 4, 8, 9, 11, or 12 wherein the one or more non-flow generator heat sources comprise any one or more of the following non-flow generator components or variables of, connected to, or associated with the apparatus: ambient temperature of environment surrounding apparatus, a battery or power source, a heater plate of a humidifier (12), a heater wire (16a) of a patient interface conduit (16), electronic circuitry or components, a Printed Circuit Board (PCB), a graphical user interface, and/or a display screen (14).

14. A respiratory support apparatus according to any one of claims 1-13 wherein the steady-state temperature is configurable to represent any predictable temperature of interest of, within, or associated with the apparatus (10); and/or wherein the steady-state temperature represents a sustained and/or stable temperature parameter, as opposed to a transient temperature parameter; and/or wherein the steady-state temperature represents a predicted temperature parameter after the apparatus (10) has been operating in the desired therapy session for at least a predetermined minimum time period at the set flow rate such that the steady-state temperature represents a sustained and/or stable temperature parameter.

15. A respiratory support apparatus according to any one of claims 1-14 wherein the steady-state temperature of the apparatus (10) represents the predicted temperature of the flow of gases in the apparatus (10) at a specified region or location within a gases flow path of the apparatus (10); and/or wherein the steady-state temperature of the apparatus (10) represents the predicted temperature within a specific location or region of a main housing (100) of the apparatus (10); and/or wherein the steady-state temperature of the apparatus (10) represents the predicted temperature at or in the region of a specific component or componentry of the respiratory support apparatus (10); and/or wherein the steady-state temperature of the apparatus (10) represents a predicted generic overall temperature parameter associated with the apparatus (10) based on a heat model of the apparatus (10) and/or one or more heat sources of interest of or associated with the apparatus (10).

16. A respiratory support apparatus according to any one of claims 1-15 wherein the high-temperature-condition threshold is a temperature parameter that is predetermined or selected based on the characteristics of the apparatus (10) and/or the specific type of steady-state temperature being predicted; and optionally: wherein the steady-state temperature represents a predicted temperature parameter relating to the temperature of flow of gases at a specified region or location within the apparatus (10), and the high-temperature-condition threshold is configured to represent a gases temperature safety threshold or value above which there is an increased risk of faulty operation, damage to the apparatus (10), and/or a safety risk to the user; and/or wherein the steady-state temperature represents a predicted temperature parameter relating to a specific location or region of a main housing (100) of the apparatus (10), and the high-temperature-condition threshold is configured to represent a housing temperature safety threshold or value above which there is an increased risk of faulty operation, damage to the apparatus (10), and/or a safety risk to the user.

17. A respiratory support apparatus according to any one of claims 1-16 wherein the controller (13) is configured to generate an alarm if a predicted-high-temperature-condition signal is generated, and optionally wherein:

the alarm comprises any one of more of the following types of alarms: audible, visual, tactile, and/or haptic; and/or the alarm comprises an alarm message or indication on a display screen (14) or a user interface of the apparatus.

18. A respiratory support apparatus according to claim 17 wherein the controller (13) is configured to reject the set flow rate and disable the motor or reduce the motor to an idling speed if the alarm is generated; or wherein the controller (13) is configured to cap the motor speed to a maximum allowable motor speed and to generate an alarm message or indication on a display screen (14) or a user interface of the apparatus (10) if an alarm is generated, and wherein the maximum allowable motor speed represents the highest motor speed, for the set flow rate, that the apparatus (10) can sustain without the steady-state temperature exceeding the high-temperature-condition threshold.

19. A respiratory support apparatus according to any one of claims 1-18 wherein the flow generator (11) comprises one or more thermistors located within the housing of the flow generator (11) or in thermal communication with the flow generator (11) or a motor of the flow generator (11), and wherein the thermistor(s) are configured to sense a temperature of or relating to the flow generator (11) and/or motor of the flow generator (11), and generate representative temperature signal(s).

**Patentansprüche**

1. Atemunterstützungsvorrichtung (10), die konfiguriert ist, um einem Benutzer einen Gasstrom für eine Atemflusstherapie bereitzustellen, umfassend:

einen Strömungserzeuger (11), der betriebsfähig ist, um einen Gasstrom zu erzeugen,
eine Steuerung (13), die betriebsmäßig mit dem Strömungserzeuger (11) verbunden ist und betriebsfähig ist, um eine Strömungsgeschwindigkeit des Gasstroms durch Steuern einer Motordrehzahl des Strömungserzeugers (11) zu steuern, wobei die Steuerung (13) konfiguriert ist zum:

Empfangen (202) einer Eingabe, die eine eingestellte Strömungsgeschwindigkeit darstellt, mit der die Vorrichtung den Gasstrom für eine gewünschte Therapiesitzung, die eine Atemflusstherapie umfasst, erzeugen soll;
Vorhersagen (204), mindestens teilweise basierend auf einer Motordrehzahl, die erforderlich ist, um die eingestellte Strömungsgeschwindigkeit zu erzeugen, ob eine Beharrungstemperatur der Vorrichtung einen Hochtemperaturzustandsschwellenwert überschreiten wird; und
Erzeugen (206) eines Signals für einen vorhergesagten Hochtemperaturzustand, wenn der Hochtemperaturzustandsschwellenwert basierend auf der Vorhersage überschritten wird.

2. Atemunterstützungsvorrichtung nach Anspruch 1, wobei das Vorhersagen (204), ob die Beharrungstemperatur den Hochtemperaturzustandsschwellenwert überschreiten wird, auf der Bestimmung basiert, ob die erforderliche Motordrehzahl eine maximal zulässige Motordrehzahl überschreitet, wobei die maximal zulässige Motordrehzahl die höchste Motordrehzahl für die eingestellte Strömungsgeschwindigkeit darstellt, die die Vorrichtung aufrechterhalten kann, ohne dass die Beharrungstemperatur den Hochtemperaturzustandsschwellenwert überschreitet.

3. Atemunterstützungsvorrichtung nach Anspruch 2, wobei die maximal zulässige Motordrehzahl auf einem vorbestimmten Wärmemodell basiert oder davon abhängig ist, und optional wobei:

das Wärmemodell die von dem Strömungserzeuger (11) erzeugte Wärme darstellt, welche von der Strömungsgeschwindigkeit und der Motordrehzahl abhängig ist; oder
das Wärmemodell die von dem Strömungserzeuger (11) erzeugte Wärme, welche von der Strömungsgeschwindigkeit und der Motordrehzahl abhängig ist, und die von einer oder mehreren Nicht-Strömungserzeuger-Wärmequellen erzeugte Wärme darstellt.

4. Atemunterstützungsvorrichtung nach Anspruch 3, wobei das Wärmemodell konfiguriert ist, um die eine oder die mehreren Nicht-Strömungserzeuger-Wärmequellen mit jeweiligen voreingestellten Werten darzustellen, die eine Schätzung der maximalen Wärme darstellen, die von jeder der einen oder mehreren Nicht-Strömungserzeuger-Wärmequellen während des Betriebs erzeugt werden könnte.

5. Atemunterstützungsvorrichtung nach Anspruch 1, wobei das Vorhersagen (204), ob die Beharrungstemperatur den Hochtemperaturzustandsschwellenwert überschreiten wird, auf Folgendem basiert:

dem Vorhersagen der Beharrungstemperatur in Abhängigkeit von der erforderlichen Motordrehzahl und der eingestellten Strömungsgeschwindigkeit; und
dem Bestimmen, ob die vorhergesagte Beharrungstemperatur den Hochtemperaturzustandsschwellenwert überschreitet, und optional wobei das Vorhersagen der Beharrungstemperatur das Bestimmen einer Schätzung aus einem vorbestimmten Wärmemodell umfasst.

6. Atemunterstützungsvorrichtung nach Anspruch 1, wobei die Steuerung (13) konfiguriert ist, um vorherzusagen (204), ob die Beharrungstemperatur der Vorrichtung den Hochtemperaturzustandsschwellenwert überschreiten wird, durch:
Bestimmen, ob die erforderliche Motordrehzahl eine maximal zulässige Motordrehzahl für die eingestellte Strömungsgeschwindigkeit überschreitet, wobei die maximal zulässige Motordrehzahl den Hochtemperaturzustandsschwellenwert darstellt, und optional wobei die maximal zulässige Motordrehzahl für die eingestellte Strömungsgeschwindigkeit basierend auf einem vorbestimmten Wärmemodell bestimmt wird oder davon abhängig ist.

7. Atemunterstützungsvorrichtung nach Anspruch 1, wobei die Steuerung (13) konfiguriert ist, um vorherzusagen (204), ob die Beharrungstemperatur der Vorrichtung den Hochtemperaturzustandsschwellenwert überschreiten wird, durch:

Vorhersagen der Beharrungstemperatur basierend auf oder in Abhängigkeit von einem vorbestimmten Wärmemodell, der eingestellten Strömungsgeschwindigkeit und der erforderlichen Motordrehzahl; und
Bestimmen, ob die vorhergesagte Beharrungstemperatur den Hochtemperaturzustandsschwellenwert überschreitet.

8. Atemunterstützungsvorrichtung nach Anspruch 7, wobei das Wärmemodell eine Temperaturvariable erzeugt, die die vorhergesagte Beharrungstemperatur darstellt, die mindestens auf der eingestellten Strömungsgeschwindigkeit, der erforderlichen Motordrehzahl und einem oder mehreren anderen Parametern basiert, die Nicht-Strömungserzeuger-Wärmequellen der Vorrichtung (10) oder der Vorrichtung zugeordnete Wärmequellen darstellen, welche zur Beharrungstemperatur der Vorrichtung (10) beitragen werden.

9. Atemunterstützungsvorrichtung nach einem der Ansprüche 3 bis 8, wobei das Wärmemodell eine oder mehrere Wärmequellen der Vorrichtung oder der Vorrichtung zugeordnete Wärmequellen darstellt, einschließlich mindestens das Darstellen der vom Strömungserzeuger (11) erzeugten Wärme in Abhängigkeit von der Motordrehzahl und der Strömungsgeschwindigkeit, und optional wobei das Wärmemodell auch die Wärme darstellt, die von einer oder mehreren Nicht-Strömungserzeuger-Wärmequellen der Vorrichtung oder von der Vorrichtung zugeordneten Nicht-Strömungserzeuger-Wärmequellen erzeugt wird, welche zur Beharrungstemperatur der Vorrichtung (10) beitragen werden.

10. Atemunterstützungsvorrichtung nach einem der Ansprüche 1 bis 9, wobei die Steuerung (13) konfiguriert ist, um die Eingabe der eingestellten Strömungsgeschwindigkeit für die gewünschte Therapiesitzung zu empfangen, wenn sich die Vorrichtung (10) in der Start- oder Bereitschaftsphase befindet, wobei der Strömungserzeuger gestoppt ist oder mit einer Nicht-Therapie-Drehzahl im Leerlauf läuft, bevor die gewünschte Therapiesitzung beginnt; und/oder wobei die Steuerung (13) konfiguriert ist, um die Eingabe der eingestellten Strömungsgeschwindigkeit zu empfangen, während die Vorrichtung (10) bereits in einer aktuellen Therapiesitzung mit einer ersten Strömungsgeschwindigkeit arbeitet, und die Eingabe der eingestellten Strömungsgeschwindigkeit eine andere zweite Strömungsgeschwindigkeit ist, die sich auf die neue gewünschte Therapiesitzung bezieht.

11. Atemunterstützungsvorrichtung nach Anspruch 1, wobei die Steuerung (13) konfiguriert ist, um mindestens teilweise basierend auf der erforderlichen Motordrehzahl vorherzusagen, ob die Beharrungstemperatur der Vorrichtung (10) den Hochtemperaturzustandsschwellenwert überschreiten wird, durch:

Schätzen eines ersten Wärmewerts, der die Wärme darstellt, die zu der Beharrungstemperatur durch eine oder mehrere Nicht-Strömungserzeuger-Wärmequellen beiträgt;
Schätzen einer maximal zulässigen Motordrehzahl des Strömungserzeugers (11), die einem zweiten Wärmewert entspricht, der die Wärme darstellt, die zu der Beharrungstemperatur durch den Strömungserzeuger (11) beiträgt, derart, dass das Aggregat des ersten Wärmewerts und des zweiten Wärmewerts nicht dazu führt, dass die Beharrungstemperatur den Hochtemperaturzustandsschwellenwert überschreitet;
Erhöhen oder Ändern der Motordrehzahl des Strömungserzeugers (11) auf die Motordrehzahl, die erforderlich ist, damit der Strömungserzeuger die eingestellte Strömungsgeschwindigkeit erzeugen kann, um dadurch die erforderliche Motordrehzahl für diese eingestellte Strömungsgeschwindigkeit zu bestimmen; und
Vorhersagen, dass die Beharrungstemperatur der Vorrichtung (10) den Hochtemperaturzustandsschwellenwert überschreiten wird, wenn die bestimmte erforderliche Motordrehzahl über der geschätzten maximal zulässigen Motordrehzahl liegt.

12. Atemunterstützungsvorrichtung nach Anspruch 1, wobei die Steuerung (13) konfiguriert ist, um mindestens teilweise basierend auf der erforderlichen Motordrehzahl vorherzusagen, ob die Beharrungstemperatur der Vorrichtung einen Hochtemperaturzustandsschwellenwert überschreiten wird, durch:

Erhöhen oder Ändern (404) der Motordrehzahl des Strömungserzeugers auf die Motordrehzahl, die erforderlich ist, damit der Strömungserzeuger die eingestellte Strömungsgeschwindigkeit erzeugen kann, um dadurch die erforderliche Motordrehzahl für diese eingestellte Strömungsgeschwindigkeit zu bestimmen;
Schätzen (406) eines ersten Beharrungstemperaturwerts, der die vom Strömungserzeuger zur Beharrungstemperatur beigetragene Wärme darstellt;
Schätzen (408) eines zweiten Beharrungstemperaturwerts, der die von einer oder mehreren Nicht-Strömungserzeuger-Wärmequellen zur Beharrungstemperatur beigetragene Wärme darstellt;
Vorhersagen (410) der Beharrungstemperatur der Vorrichtung durch Aggregieren des ersten Beharrungstemperaturwerts und des zweiten Beharrungstemperaturwerts; und
Bewerten (412), ob die vorhergesagte Beharrungstemperatur den Hochtemperaturzustandsschwellenwert überschreitet.

13. Atemunterstützungsvorrichtung nach einem der Ansprüche 3, 4, 8, 9, 11 oder 12, wobei die eine oder mehreren Nicht-Strömungserzeuger-Wärmequellen eine oder mehrere der folgenden Nicht-Strömungserzeuger-Komponenten oder -Variablen der Vorrichtung, bzw. die mit der Vorrichtung verbunden oder der Vorrichtung zugeordnet sind, umfassen: Umgebungstemperatur der die Vorrichtung umgebenden Umgebung, eine Batterie oder Energiequelle, eine Heizplatte eines Befeuchters (12), einen Heizdraht (16a) einer Patientenschnittstellenleitung (16), elektronische Schaltkreise oder Komponenten, eine Leiterplatte (PCB), eine grafische Benutzerschnittstelle und/oder einen Anzeigebildschirm (14).

14. Atemunterstützungsvorrichtung nach einem der Ansprüche 1 bis 13, wobei die Beharrungstemperatur so konfigurierbar ist, dass sie eine beliebige vorhersagbare Temperatur von Interesse der Vorrichtung (10), bzw. in derselben oder derselben zugeordnet, darstellt; und/oder wobei die Beharrungstemperatur einen anhaltenden und/oder stabilen Temperaturparameter darstellt, im Gegensatz zu einem transienten Temperaturparameter; und/oder wobei die Beharrungstemperatur einen vorhergesagten Temperaturparameter darstellt, nachdem die Vorrichtung (10) in der gewünschten Therapiesitzung für mindestens eine vorbestimmte minimale Zeitspanne bei der eingestellten Strömungsgeschwindigkeit betrieben wurde, sodass die Beharrungstemperatur einen anhaltenden und/oder stabilen Temperaturparameter darstellt.

15. Atemunterstützungsvorrichtung nach einem der Ansprüche 1 bis 14, wobei die Beharrungstemperatur der Vorrichtung (10) die vorhergesagte Temperatur des Gasstroms in der Vorrichtung (10) an einem bestimmten Bereich oder einer bestimmten Stelle innerhalb eines Gasströmungswegs der Vorrichtung (10) darstellt; und/oder wobei die Beharrungstemperatur der Vorrichtung (10) die vorhergesagte Temperatur innerhalb einer bestimmten Stelle oder eines bestimmten Bereichs eines Hauptgehäuses (100) der Vorrichtung (10) darstellt; und/oder wobei die Beharrungstemperatur der Vorrichtung (10) die vorhergesagte Temperatur an oder in dem Bereich einer bestimmten Komponente oder Komponentenanordnung der Atemunterstützungsvorrichtung (10) darstellt; und/oder wobei die Beharrungstemperatur der Vorrichtung (10) einen vorhergesagten generischen Gesamttemperaturparameter darstellt, der der Vorrichtung (10) zugeordnet ist, basierend auf einem Wärmemodell der Vorrichtung (10) und/oder einer oder mehreren Wärmequellen von Interesse der Vorrichtung (10) oder dieser zugeordnet.

16. Atemunterstützungsvorrichtung nach einem der Ansprüche 1 bis 15, wobei der Hochtemperaturzustandsschwellenwert ein Temperaturparameter ist, der basierend auf den Eigenschaften der Vorrichtung (10) und/oder der spezifischen Art der vorhergesagten Beharrungstemperatur vorbestimmt oder ausgewählt wird; und optional: wobei die Beharrungstemperatur einen vorhergesagten Temperaturparameter darstellt, der sich auf die Temperatur des Gasstroms an einem bestimmten Bereich oder einer bestimmten Stelle innerhalb der Vorrichtung (10) bezieht, und der Hochtemperaturzustandsschwellenwert konfiguriert ist, um einen Gastemperatur-Sicherheitsschwellenwert oder -wert darzustellen, oberhalb dessen ein erhöhtes Risiko für fehlerhaften Betrieb, Schäden an der Vorrichtung (10) und/oder ein Sicherheitsrisiko für den Benutzer besteht; und/oder wobei die Beharrungstemperatur einen vorhergesagten Temperaturparameter darstellt, der sich auf eine bestimmte Stelle oder einen bestimmten Bereich eines Hauptgehäuses (100) der Vorrichtung (10) bezieht, und der Hochtemperaturzustandsschwellenwert konfiguriert ist, um einen Gehäusetemperatur-Sicherheitsschwellenwert oder -wert darzustellen, oberhalb dessen ein erhöhtes Risiko für fehlerhaften Betrieb, Schäden an der Vorrichtung (10) und/oder ein Sicherheitsrisiko für den Benutzer besteht.

17. Atemunterstützungsvorrichtung nach einem der Ansprüche 1 bis 16, wobei die Steuerung (13) konfiguriert ist, um einen Alarm zu erzeugen, wenn ein Signal für einen vorhergesagten Hochtemperaturzustand erzeugt wird, und optional wobei:

  der Alarm einen oder mehrere der folgenden Alarmtypen umfasst: hörbar, visuell, taktil und/oder haptisch; und/oder
  der Alarm eine Alarmmeldung oder Anzeige auf einem Anzeigebildschirm (14) oder einer Benutzerschnittstelle der Vorrichtung umfasst.

18. Atemunterstützungsvorrichtung nach Anspruch 17, wobei die Steuerung (13) konfiguriert ist, um die eingestellte Strömungsgeschwindigkeit zu verwerfen und den Motor zu deaktivieren oder den Motor auf eine Leerlaufdrehzahl zu reduzieren, wenn der Alarm erzeugt wird; oder wobei die Steuerung (13) konfiguriert ist, um die Motordrehzahl auf eine maximal zulässige Motordrehzahl zu begrenzen und eine Alarmmeldung oder -anzeige auf einem Anzeigebildschirm (14) oder einer Bedienerschnittstelle der Vorrichtung (10) zu erzeugen, wenn ein Alarm erzeugt wird, und wobei die maximal zulässige Motordrehzahl die höchste Motordrehzahl für die eingestellte Strömungsgeschwindigkeit darstellt, die die Vorrichtung (10) aufrechterhalten kann, ohne dass die Beharrungstemperatur den Hochtemperaturzustandsschwellenwert überschreitet.

**19.** Atemunterstützungsvorrichtung nach einem der Ansprüche 1 bis 18, wobei der Strömungserzeuger (11) einen oder mehrere Thermistoren umfasst, die innerhalb des Gehäuses des Strömungserzeugers (11) oder in thermischer Verbindung mit dem Strömungserzeuger (11) oder einem Motor des Strömungserzeugers (11) angeordnet sind, und worin der/die Thermistor(en) konfiguriert sind, eine Temperatur des Strömungserzeugers (11) und/oder Motors des Strömungserzeugers (11) oder eine damit in Zusammenhang stehende Temperatur zu erfassen und repräsentative Temperatursignal(e) zu erzeugen.

**Revendications**

**1.** Appareil (10) d'assistance respiratoire qui est conçu pour fournir un écoulement de gaz à un utilisateur pour une thérapie par écoulement respiratoire comprenant :

un générateur d'écoulement (11) qui est fonctionnel pour générer un écoulement de gaz ;
une unité de commande (13) qui est fonctionnellement connectée au générateur d'écoulement (11) et fonctionnelle pour commander un débit d'écoulement de l'écoulement de gaz en commandant une vitesse de moteur du générateur d'écoulement (11), dans lequel l'unité de commande (13) est configurée pour :

recevoir (202) une entrée qui représente un débit d'écoulement réglé auquel l'appareil doit générer l'écoulement de gaz pour une séance de thérapie souhaitée comprenant une thérapie par écoulement respiratoire ;
prédire (204) si une température en régime permanent de l'appareil va dépasser un seuil de condition de haute température, sur la base au moins partiellement d'une vitesse de moteur requise pour générer le débit d'écoulement réglé ; et
générer (206) un signal de condition de haute température prédite si le seuil de condition de haute température est dépassé sur la base de la prédiction.

**2.** Appareil d'assistance respiratoire selon la revendication 1 dans lequel le fait de prédire (204) si la température en régime permanent va dépasser le seuil de condition de haute température est sur la base de la détermination quant à savoir si la vitesse de moteur requise dépasse une vitesse maximale admissible de moteur, dans lequel la vitesse maximale admissible de moteur représente la vitesse de moteur la plus élevée, pour le débit d'écoulement réglé, que l'appareil peut maintenir sans que la température en régime permanent ne dépasse le seuil de condition de haute température.

**3.** Appareil d'assistance respiratoire selon la revendication 2 dans lequel la vitesse maximale admissible de moteur est sur la base, ou est une fonction, d'un modèle thermique prédéterminé, et facultativement dans lequel :

le modèle thermique représente la chaleur générée par le générateur d'écoulement (11), qui est une fonction du débit d'écoulement et de la vitesse de moteur ; ou
le modèle thermique représente la chaleur générée par le générateur d'écoulement (11), qui est une fonction du débit d'écoulement et de la vitesse de moteur, et d'une ou plusieurs sources de chaleur non génératrices d'écoulement.

**4.** Appareil d'assistance respiratoire selon la revendication 3 dans lequel le modèle thermique est configuré pour représenter la ou les sources de chaleur non génératrices d'écoulement avec des valeurs préréglées respectives représentant une estimation de la chaleur maximale qui pourrait être générée par chacune parmi la ou les sources de chaleur non génératrices d'écoulement pendant le fonctionnement.

**5.** Appareil d'assistance respiratoire selon la revendication 1 dans lequel le fait de prédire (204) si la température en régime permanent va dépasser le seuil de condition de haute température est sur la base de :

la prédiction de la température en régime permanent en fonction de la vitesse de moteur requise et du débit d'écoulement réglé ; et
le fait de déterminer si la température en régime permanent prédite dépasse le seuil de condition de haute température, et facultativement dans lequel la prédiction de la température en régime permanent comprend la détermination d'une estimation à partir d'un modèle thermique prédéterminé.

**6.** Appareil d'assistance respiratoire selon la revendication 1 dans lequel l'unité de commande (13) est configurée pour prédire (204) si la température en régime permanent de l'appareil va dépasser le seuil de condition de haute température par :

le fait de déterminer si la vitesse de moteur requise dépasse une vitesse maximale admissible de moteur pour le débit d'écoulement réglé, la vitesse maximale admissible de moteur représentant le seuil de condition de haute température, et facultativement dans lequel la vitesse maximale admissible de moteur pour le débit d'écoulement réglé est déterminée sur la base, ou est une fonction, d'un modèle thermique prédéterminé.

**7.** Appareil d'assistance respiratoire selon la revendication 1 dans lequel l'unité de commande (13) est configurée pour prédire (204) si la température en régime permanent de l'appareil va dépasser le seuil de condition de haute température par :

la prédiction de la température en régime permanent sur la base, ou en fonction, d'un modèle thermique prédéterminé, du débit d'écoulement réglé, et de la vitesse de moteur requise ; et
le fait de déterminer si la température en régime permanent prédite dépasse le seuil de condition de haute température.

**8.** Appareil d'assistance respiratoire selon la revendication 7 dans lequel le modèle thermique génère une variable de température représentant la température en régime permanent prédite sur la base au moins du débit d'écoulement réglé, de la vitesse de moteur requise et d'un ou plusieurs autres paramètres représentant des sources de chaleur non génératrices d'écoulement de, ou associées à, l'appareil (10) qui vont contribuer à la température en régime permanent de l'appareil (10).

**9.** Appareil d'assistance respiratoire selon l'une quelconque des revendications 3 à 8 dans lequel le modèle thermique représente une ou plusieurs sources de chaleur de, ou associées à, l'appareil, comportant au moins la représentation de la chaleur générée par le générateur d'écoulement (11) en fonction de la vitesse de moteur et du débit d'écoulement, et facultativement dans lequel le modèle thermique représente en outre la chaleur générée par une ou plusieurs sources de chaleur non génératrices d'écoulement de, ou associées à, l'appareil, qui vont contribuer à la température en régime permanent de l'appareil (10).

**10.** Appareil d'assistance respiratoire selon l'une quelconque des revendications 1 à 9 dans lequel l'unité de commande (13) est configurée pour recevoir l'entrée de débit d'écoulement réglé pour la séance de thérapie souhaitée lorsque l'appareil (10) est au démarrage ou en attente avec le générateur d'écoulement arrêté ou au ralenti à une vitesse non thérapeutique avant le commencement de la séance de thérapie souhaitée ; et/ou dans lequel l'unité de commande (13) est configurée pour recevoir l'entrée de débit d'écoulement réglé alors que l'appareil (10) fonctionne déjà dans une séance de thérapie actuelle à un premier débit d'écoulement, et l'entrée de débit d'écoulement réglé est un second débit d'écoulement différent se rapportant à la nouvelle séance de thérapie souhaitée.

**11.** Appareil d'assistance respiratoire selon la revendication 1 dans lequel l'unité de commande (13) est configurée pour prédire si la température en régime permanent de l'appareil (10) va dépasser le seuil de condition de haute température sur la base au moins partiellement de la vitesse de moteur requise par :

l'estimation d'une première valeur de chaleur représentant la chaleur contribuant à la température en régime permanent par une ou plusieurs sources de chaleur non génératrices d'écoulement ;
l'estimation d'une vitesse maximale admissible de moteur du générateur d'écoulement (11) correspondant à une seconde valeur de chaleur représentant la chaleur contribuant à la température en régime permanent par le générateur d'écoulement (11) de telle sorte que le total de la première valeur de chaleur et de la seconde valeur de chaleur n'amène pas la température en régime permanent à dépasser le seuil de condition de haute température ;
l'augmentation ou le changement de la vitesse de moteur du générateur d'écoulement (11) à la vitesse de moteur requise pour que le générateur d'écoulement génère le débit d'écoulement réglé de façon à déterminer de ce fait la vitesse de moteur requise pour ce débit d'écoulement réglé ; et
la prédiction que la température en régime permanent de l'appareil (10) va dépasser le seuil de condition de haute température si la vitesse de moteur requise déterminée est supérieure à la vitesse maximale admissible de moteur estimée.

**12.** Appareil d'assistance respiratoire selon la revendication 1 dans lequel l'unité de commande (13) est configurée pour prédire si une température en régime permanent de l'appareil va dépasser le seuil de condition de haute température sur la base au moins partiellement de la vitesse de moteur requise par :

l'augmentation ou le changement (404) de la vitesse de moteur du générateur d'écoulement à la vitesse de moteur requise pour que le générateur d'écoulement génère le débit d'écoulement réglé de façon à déterminer de ce fait la vitesse de moteur requise pour ce débit d'écoulement réglé ;

l'estimation (406) d'une première valeur de température en régime permanent représentant la chaleur contribuant à la température en régime permanent par le générateur d'écoulement ;

l'estimation (408) d'une seconde valeur de température en régime permanent représentant la chaleur contribuant à la température en régime permanent par une ou plusieurs sources de chaleur non génératrices d'écoulement ;

la prédiction (410) de la température en régime permanent de l'appareil en totalisant la première valeur de température en régime permanent et la seconde valeur de température en régime permanent ; et

le fait d'évaluer (412) si la température en régime permanent prédite dépasse le seuil de condition de haute température.

13. Appareil d'assistance respiratoire selon l'une quelconque des revendications 3, 4, 8, 9, 11 ou 12 dans lequel la ou les sources de chaleur non génératrices d'écoulement comprennent l'un quelconque ou plusieurs quelconques des composants non générateurs d'écoulement, ou variables de ceux-ci, suivants, connectés, ou associés, à l'appareil : température ambiante de l'environnement entourant l'appareil, une batterie ou une source de puissance, une plaque chauffante d'un humidificateur (12), un fil chauffant (16a) d'un conduit d'interface patient (16), un système de circuits ou des composants électroniques, une carte de circuit imprimé (PCB), une interface utilisateur graphique et/ou un écran d'affichage (14).

14. Appareil d'assistance respiratoire selon l'une quelconque des revendications 1 à 13 dans lequel la température en régime permanent est configurable pour représenter l'une quelconque température d'intérêt prévisible de, au sein de, ou associée à, l'appareil (10) ; et/ou dans lequel la température en régime permanent représente un paramètre de température maintenu et/ou stable, par opposition à un paramètre de température transitoire ; et/ou dans lequel la température en régime permanent représente un paramètre de température prédit après que l'appareil (10) a été mis en fonctionnement dans la séance de thérapie souhaitée pendant au moins un laps de temps minimal prédéterminé au débit d'écoulement réglé de telle sorte que la température en régime permanent représente un paramètre de température maintenu et/ou stable.

15. Appareil d'assistance respiratoire selon l'une quelconque des revendications 1 à 14 dans lequel la température en régime permanent de l'appareil (10) représente la température prédite de l'écoulement de gaz dans l'appareil (10) au niveau d'une région ou localisation spécifiée au sein d'un trajet d'écoulement de gaz de l'appareil (10) ; et/ou dans lequel la température en régime permanent de l'appareil (10) représente la température prédite au sein d'une localisation ou région spécifique d'un logement principal (100) de l'appareil (10) ; et/ou dans lequel la température en régime permanent de l'appareil (10) représente la température prédite au niveau de ou dans la région d'un composant ou système de composants spécifique de l'appareil (10) d'assistance respiratoire ; et/ou dans lequel la température en régime permanent de l'appareil (10) représente un paramètre de température global générique prédit associé à l'appareil (10) sur la base d'un modèle thermique de l'appareil (10) et/ou d'une ou plusieurs sources de chaleur d'intérêt de, ou associées à, l'appareil (10).

16. Appareil d'assistance respiratoire selon l'une quelconque des revendications 1 à 15 dans lequel le seuil de condition de haute température est un paramètre de température qui est prédéterminé ou sélectionné sur la base des caractéristiques de l'appareil (10) et/ou du type spécifique de température en régime permanent étant prédit ; et facultativement : dans lequel la température en régime permanent représente un paramètre de température prédit se rapportant à la température d'écoulement de gaz au niveau d'une région ou localisation spécifiée au sein de l'appareil (10), et le seuil de condition de haute température est configuré pour représenter un seuil de sécurité de température de gaz ou une valeur au-dessus de laquelle il y a un risque accru de fonctionnement défectueux, de dommage à l'appareil (10), et/ou un risque de sécurité pour l'utilisateur ; et/ou dans lequel la température en régime permanent représente un paramètre de température prédit se rapportant à une localisation ou région spécifique d'un logement principal (100) de l'appareil (10), et le seuil de condition de haute température est configuré pour représenter un seuil de sécurité de température de logement ou une valeur au-dessus de laquelle il y a un risque accru de fonctionnement défectueux, de dommage à l'appareil (10) et/ou un risque de sécurité pour l'utilisateur.

17. Appareil d'assistance respiratoire selon l'une quelconque des revendications 1 à 16 dans lequel l'unité de commande (13) est configurée pour générer une alarme si un signal de condition de haute température prédite est généré, et facultativement dans lequel :

l'alarme comprend l'un quelconque ou plusieurs quelconques parmi les types d'alarmes suivants : audible, visuelle, tactile et/ou haptique ; et/ou

l'alarme comprend un message d'alarme ou une indication sur un écran d'affichage (14) ou une interface utilisateur de l'appareil.

18. Appareil d'assistance respiratoire selon la revendication 17 dans lequel l'unité de commande (13) est configurée pour rejeter le débit d'écoulement réglé et désactiver le moteur ou faire descendre le moteur à une vitesse de ralenti si l'alarme est générée ; ou dans lequel l'unité de commande (13) est configurée pour limiter la vitesse de moteur à une vitesse maximale admissible de moteur et pour générer un message d'alarme ou une indication sur un écran d'affichage (14) ou une interface utilisateur de l'appareil (10) si une alarme est générée, et dans lequel la vitesse maximale admissible de moteur représente la vitesse de moteur la plus élevée, pour le débit d'écoulement réglé, que l'appareil (10) peut maintenir sans que la température en régime permanent ne dépasse le seuil de condition de haute température.

19. Appareil d'assistance respiratoire selon l'une quelconque des revendications 1 à 18 dans lequel le générateur d'écoulement (11) comprend une ou plusieurs thermistances localisées au sein du logement du générateur d'écoulement (11) ou en communication thermique avec le générateur d'écoulement (11) ou un moteur du générateur d'écoulement (11), et dans lequel la ou les thermistance(s) sont configurées pour capter une température du, ou se rapportant au, générateur d'écoulement (11) et/ou moteur du générateur d'écoulement (11), et générer un ou des signal/signaux de température représentatif(s).

**Figure 1A**

**Figure 1B**

200

202
Receive input setting(s) for a new respiratory therapy session

204
Predict if steady-state temperature of apparatus will exceed high-temperature-condition threshold

206
Generate a predicted-high-temperature-condition signal or alarm signal if high-temperature-condition threshold exceeded based on the prediction

208
Activate alarm responses in response to high-temperature-condition or alarm signal.

Figure 2

*300*

*302*

Receive set flow rate input

*304*

Estimate a heat value for non-blower heat sources

*306*

Estimate a maximum allowable motor speed for set flow rate such that the total heat from the blower and non-blower heat sources will not cause a high-temperature-condition in the steady-state temperature

*308*

Change or increase motor speed of blower to speed required to achieve the set flow rate

*310*

Trigger predictive alarm if required motor speed exceeds the maximum allowable motor speed

Figure 3

**Figure 4A**

**Figure 4B**

The processor predicts that the steady-state temperature of the device **will exceed** $T_{max}$ during therapy.

For the current flow, is the motor speed above the $\omega_{max}$ curve? — 320

Yes

No

The processor predicts that the steady-state temperature of the device **will not exceed** $T_{max}$ during therapy.

The processor immediately activates the maximum-device-temperature alarm. — 322

The processor does not activate the maximum-device-temperature alarm at this time. — 324

**Figure 4C**

Figure 5

Figure 6A

430 — Is $T_1 + \Delta T > T_{max}$?

Yes — The processor predicts that the steady-state temperature of the device **will exceed** $T_{max}$ during therapy.

432 — The processor immediately activates the maximum-device-temperature alarm.

No — The processor predicts that the steady-state temperature of the device **will not exceed** $T_{max}$ during therapy.

434 — The processor does not activate the maximum-device-temperature alarm at this time.

Figure 6B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080163872
- WO 2017200394 A
- WO 2018052320 A
- WO 2017095241 A
- WO 2016207838 A